# EUROPEAN PATENT SPECIFICATION

(11) **EP 1 646 657 B1**
(45) Date of publication and mention of the grant of the patent: **16.11.2011**
(21) Application number: 04744979.8
(22) Date of filing: 15.07.2004
(51) Int. Cl.: C07K 16/28, A61K 39/395, G01N 33/53, G01N 33/577, C12N 15/13, C12N 5/20, A61P 37/00

(54) **ANTIBODIES TO CD44 VRA AND METHODS OF USE**
ANTIKÖRPER GEGEN CD44VRA UND METHODEN ZU IHRER VERWENDUNG
ANTICORPS ANTI-CD44VRA ET LEURS PROCEDES D'UTILISATION

(30) Priority: 15.07.2003 US 486919 P; 19.08.2003 US 495876 P
(43) Date of publication of application: 19.04.2006
(73) Proprietor: Yissum Research Development Company of the Hebrew University of Jerusalem Ltd., 91390 Jerusalem (IL)
(72) Inventor: NAOR, David, 90830 Bait Nekuffa (IL); GOLAN, Itshak, 77632 Ashdod (IL)
(74) Representative: Machtalère, Georges
(86) International application number: PCT/IL2004/000639
(87) International publication number: WO 2005/007700

(56) References cited:
- WO-A-00/75312
- WO-A-03/014160
- WO-A-03/072606
- NEDVETZKI SHLOMO ET AL: "A mutation in a CD44 variant of inflammatory cells enhances the mitogenic interaction of FGF with its receptor." JOURNAL OF CLINICAL INVESTIGATION, vol. 111, no. 8, April 2003 (2003-04), pages 1211-1220, XP001152920 ISSN: 0021-9738

### SEQUENCE LISTING

<110> YISSUM RESEARCH DEVELOPMENT COMPANY OF THE HEBREW UNIVERSITY OF JERUSALEM
<120> ANTIBODIES TO CD44RA VARIANT AND METHODS OF USE
<130> 1533587
<150> US 60/486,919
   <151> 2003-07-15
<150> US 60/495,876
   <151> 2003-08-19
<160> 14
<170> PatentIn version 3.1
<210> 1
   <211> 2100
   <212> DNA
   <213> HUMAN
<400> 1
   atggacaagt tttggtggca cgcagcctgg ggactctgcc tcgtgccgct gagcctggcg    60
   cagatcgatt tgaatataac ctgccgcttt gcaggtgtat tccacgtgga gaaaaatggt   120
   cgctacagca tctctcggac ggaggccgct gacctctgca aggctttcaa tagcaccttg   180
   cccacaatgg cccagatgga gaaagctctg agcatcggat ttgagacctg caggtatggg   240
   ttcatagaag ggcacgtggt gattccccgg atccacccca actccatctg tgcagcaaac   300
   aacacagggg tgtacatcct cacatccaac acctcccagt atgacacata ttgcttcaat   360
   gcttcagctc cacctgaaga agattgtaca tcagtcacag acctgcccaa tgcctttgat   420
   ggaccaatta ccataactat tgttaaccgt gatggcaccc gctatgtcca gaaaggagaa   480
   tacagaacga atcctgaaga catctacccc agcaacccta ctgatgatga cgtgagcagc   540
   ggctcctcca gtgaaaggag cagcacttca ggaggttaca tcttttacac cttttctact   600
   gtacacccca tcccagacga agacagtccc tggatcaccg acagcacaga cagaatccct   660
   gctaccagta cgtcttcaaa taccatctca gcaggctggg agccaaatga agaaaatgaa    720
   gatgaaagag acagacacct cagtttttct ggatcaggca ttgatgatga tgaagatttt    780
   atctccagca ccatttcaac cacaccacgg gcttttgacc acacaaaaca gaaccaggac    840
   tggacccagt ggaacccaag ccattcaaat ccggaagtgc tacttcagac aaccacaagg    900
   atgactgcag atgtagacag aaatggcacc actgcttatg aaggaaactg gaacccagaa    960
   gcacaccctc ccctcattca ccatgagcat catgaggaag aagagacccc acattctaca   1020
   agcacaatcc aggcaactcc tagtagtaca acggaagaaa cagctaccca gaaggaacag   1080
   tggtttggca acagatggca tgagggatat cgccaaacac ccagagaaga ctcccattcg   1140
   acaacaggga cagctgcagc ctcagctcat accagccatc caatgcaagg aaggacaaca   1200
   ccaagcccag aggacagttc ctggactgat ttcttcaacc caatctcaca ccccatggga   1260
   cgaggtcatc aagcaggaag aaggatggat atggactcca gtcatagtac aacgcttcag   1320
   cctactgcaa atccaaacac aggtttggtg gaagatttgg acaggacagg acctctttca   1380
   atgacaacgc agcagagtaa ttctcagagc ttctctacat cacatgaagg cttggaagaa   1440
   gataaagacc atccaacaac ttctactctg acatcaagca ataggaatga tgtcacaggt   1500
   ggaagaagag acccaaatca ttctgaaggc tcaactactt tactggaagg ttatacctct   1560
   cattacccac acacgaagga aagcaggacc ttcatcccag tgacctcagc taagactggg   1620
   tcctttggag ttactgcagt tactgttgga gattccaact ctaatgtcaa tcgttcctta   1680
   tcaggagacc aagacacatt ccaccccagt ggggggtccc ataccactca tggatctgaa    1740
   tcagatggac actcacatgg gagtcaagaa ggtggagcaa acacaacctc tggtcctata   1800
   aggacacccc aaattccaga atggctgatc atcttggcat ccctcttggc cttggctttg   1860
   attcttgcag tttgcattgc agtcaacagt cgaagaaggt gtgggcagaa gaaaaagcta   1920
   gtgatcaaca gtggcaatgg agctgtggag gacagaaagc caagtggact caacggagag   1980
   gccagcaagt ctcaggaaat ggtgcatttg gtgaacaagg agtcgtcaga aactccagac   2040
   cagtttatga cagctgatga gacaaggaac ctgcagaatg tggacatgaa gattggggtg   2100
<210> 2
   <211> 700
   <212> PRT
   <213> HUMAN
<400> 2
   Met Asp Lys Phe Trp Trp His Ala Ala Trp Gly Leu Cys Leu Val Pro
   1               5                   10                  15
   Leu Ser Leu Ala Gln Ile Asp Leu Asn Ile Thr Cys Arg Phe Ala Gly
               20                  25                  30
   Val Phe His Val Glu Lys Asn Gly Arg Tyr Ser Ile Ser Arg Thr Glu
   35 40 45
   Ala Ala Asp Leu Cys Lys Ala Phe Asn Ser Thr Leu Pro Thr Met Ala
   50 55 60
   Gln Met Glu Lys Ala Leu Ser Ile Gly Phe Glu Thr Cys Arg Tyr Gly
   65 70 75 80
   Phe Ile Glu Gly His Val Val Ile Pro Arg Ile His Pro Asn Ser Ile
   85 90 95
   Cys Ala Ala Asn Asn Thr Gly Val Tyr Ile Leu Thr Ser Asn Thr Ser
   100 105 110
   Gln Tyr Asp Thr Tyr Cys Phe Asn Ala Ser Ala Pro Pro Glu Glu Asp
   115 120 125
   Cys Thr Ser Val Thr Asp Leu Pro Asn Ala Phe Asp Gly Pro Ile Thr
   130 135 140
   Ile Thr Ile Val Asn Arg Asp Gly Thr Arg Tyr Val Gln Lys Gly Glu
   145 150 155 160
   Tyr Arg Thr Asn Pro Glu Asp Ile Tyr Pro Ser Asn Pro Thr Asp Asp
   165 170 175
   Asp Val Ser Ser Gly Ser Ser Ser Glu Arg Ser Ser Thr Ser Gly Gly
   180 185 190
   Tyr Ile Phe Tyr Thr Phe Ser Thr Val His Pro Ile Pro Asp Glu Asp
   195 200 205
   Ser Pro Trp Ile Thr Asp Ser Thr Asp Arg Ile Pro Ala Thr Thr Thr
   210 215 220
   Ser Ser Asn Thr Ile Ser Ala Gly Trp Glu Pro Asn Glu Glu Asn Glu
   225 230 235 240
   Asp Glu Arg Asp Arg His Leu Ser Phe Ser Gly Ser Gly Ile Asp Asp
   245 250 255
   Asp Glu Asp Phe Ile Ser Ser Thr Ile Ser Thr Thr Pro Arg Ala Phe
   260 265 270
   Asp His Thr Lys Gln Asn Gln Asp Trp Thr Gln Trp Asn Pro Ser His
   275 280 285
   Ser Asn Pro Glu Val Leu Leu Gln Thr Thr Thr Arg Met Thr Ala Asp
   290 295 300
   Val Asp Arg Asn Gly Thr Thr Ala Tyr Glu Gly Asn Trp Asn Pro Glu
   305 310 315 320
   Ala His Pro Pro Leu Ile His His Glu His His Glu Glu Glu Glu Thr
   325 330 335
   Pro His Ser Thr Ser Thr Ile Gln Ala Thr Pro Ser Ser Thr Thr Glu
   340 345 350
   Glu Thr Ala Thr Gln Lys Glu Gln Trp Phe Gly Asn Arg Trp His Glu
   355 360 365
   Gly Tyr Arg Gln Thr Pro Arg Glu Asp Ser His Ser Thr Thr Gly Thr
   370 375 380
   Ala Ala Ala Ser Ala His Thr Ser His Pro Met Gln Gly Arg Thr Thr
   385 390 395 400
   Pro Ser Pro Glu Asp Ser Ser Trp Thr Asp Phe Phe Asn Pro Ile Ser
   405 410 415
   His Pro Met Gly Arg Gly His Gln Ala Gly Arg Arg Met Asp Met Asp
   420 425 430
   Ser Ser His Ser Thr Thr Leu Gln Pro Thr Ala Asn Pro Asn Thr Gly
   435 440 445
   Leu Val Glu Asp Leu Asp Arg Thr Gly Pro Leu Ser Met Thr Thr Gln
   450 455 460
   Gln Ser Asn Ser Gln Ser Phe Ser Thr Ser His Glu Gly Leu Glu Glu
   465 470 475 480
   Asp Lys Asp His Pro Thr Thr Ser Thr Leu Thr Ser Ser Asn Arg Asn
   485 490 495
   Asp Val Thr Gly Gly Arg Arg Asp Pro Asn His Ser Glu Gly Ser Thr
   500 505 510
   Thr Leu Leu Glu Gly Tyr Thr Ser His Tyr Pro His Thr Lys Glu Ser
   515 520 525
   Arg Thr Phe Ile Pro Val Thr Ser Ala Lys Thr Gly Ser Phe Gly Val
   530 535 540
   Thr Ala Val Thr Val Gly Asp Ser Asn Ser Asn Val Asn Arg Ser Leu
   545 550 555 560
   Ser Gly Asp Gln Asp Thr Phe His Pro Ser Gly Gly Ser His Thr Thr
   565 570 575
   His Gly Ser Glu Ser Asp Gly His Ser His Gly Ser Gln Glu Gly Gly
   580 585 590
   Ala Asn Thr Thr Ser Gly Pro Ile Arg Thr Pro Gln Ile Pro Glu Trp
   595 600 605
   Leu Ile Ile Leu Ala Ser Leu Leu Ala Leu Ala Leu Ile Leu Ala Val
   610 615 620
   Cys Ile Ala Val Asn Ser Arg Arg Arg Cys Gly Gln Lys Lys Lys Leu
   625 630 635 640
   Val Ile Asn Ser Gly Asn Gly Ala Val Glu Asp Arg Lys Pro Ser Gly
   645 650 655
   Leu Asn Gly Glu Ala Ser Lys Ser Gln Glu Met Val His Leu Val Asn
   660 665 670
   Lys Glu Ser Ser Glu Thr Pro Asp Gln Phe Met Thr Ala Asp Glu Thr
   675 680 685
   Arg Asn Leu Gln Asn Val Asp Met Lys Ile Gly Val
   690 695 700
<210> 3
   <211> 2097
   <212> DNA
   <213> HUMAN
<400> 3
   atggacaagt tttggtggca cgcagcctgg ggactctgcc tcgtgccgct gagcctggcg 60
   cagatcgatt tgaatataac ctgccgcttt gcaggtgtat tccacgtgga gaaaaatggt 120
   cgctacagca tctctcggac ggaggccgct gacctctgca aggctttcaa tagcaccttg 180
   cccacaatgg cccagatgga gaaagctctg agcatcggat ttgagacctg caggtatggg 240
   ttcatagaag ggcacgtggt gattccccgg atccacccca actccatctg tgcagcaaac 300
   aacacagggg tgtacatcct cacatccaac acctcccagt atgacacata ttgcttcaat 360
   gcttcagctc cacctgaaga agattgtaca tcagtcacag acctgcccaa tgcctttgat 420
   ggaccaatta ccataactat tgttaaccgt gatggcaccc gctatgtcca gaaaggagaa 480
   tacagaacga atcctgaaga catctacccc agcaacccta ctgatgatga cgtgagcagc 540
   ggctcctcca gtgaaaggag cagcacttca ggaggttaca tcttttacac cttttctact 600
   gtacacccca tcccagacga agacagtccc tggatcaccg acagcacaga cagaatccct 660
   gctaccagta cgtcttcaaa taccatctca gcaggctggg agccaaatga agaaaatgaa 720
   gatgaaagag acagacacct cagtttttct ggatcaggca ttgatgatga tgaagatttt     780
   atctccagca ccatttcaac cacaccacgg gcttttgacc acacaaaaca gaaccaggac     840
   tggacccagt ggaacccaag ccattcaaat ccggaagtgc tacttcagac aaccacaagg     900
   atgactgatg tagacagaaa tggcaccact gcttatgaag gaaactggaa cccagaagca     960
   caccctcccc tcattcacca tgagcatcat gaggaagaag agaccccaca ttctacaagc    1020
   acaatccagg caactcctag tagtacaacg gaagaaacag ctacccagaa ggaacagtgg    1080
   tttggcaaca gatggcatga gggatatcgc caaacaccca gagaagactc ccattcgaca    1140
   acagggacag ctgcagcctc agctcatacc agccatccaa tgcaaggaag gacaacacca    1200
   agcccagagg acagttcctg gactgatttc ttcaacccaa tctcacaccc catgggacga    1260
   ggtcatcaag caggaagaag gatggatatg gactccagtc atagtacaac gcttcagcct    1320
   actgcaaatc caaacacagg tttggtggaa gatttggaca ggacaggacc tctttcaatg    1380
   acaacgcagc agagtaattc tcagagcttc tctacatcac atgaaggctt ggaagaagat    1440
   aaagaccatc caacaacttc tactctgaca tcaagcaata ggaatgatgt cacaggtgga    1500
   agaagagacc caaatcattc tgaaggctca actactttac tggaaggtta tacctctcat    1560
   tacccacaca cgaaggaaag caggaccttc atcccagtga cctcagctaa gactgggtcc    1620
   tttggagtta ctgcagttac tgttggagat tccaactcta atgtcaatcg ttccttatca    1680
   ggagaccaag acacattcca ccccagtggg gggtcccata ccactcatgg atctgaatca    1740
   gatggacact cacatgggag tcaagaaggt ggagcaaaca caacctctgg tcctataagg    1800
   acaccccaaa ttccagaatg gctgatcatc ttggcatccc tcttggcctt ggctttgatt    1860
   cttgcagttt gcattgcagt caacagtcga agaaggtgtg ggcagaagaa aaagctagtg    1920
   atcaacagtg gcaatggagc tgtggaggac agaaagccaa gtggactcaa cggagaggcc    1980
   agcaagtctc aggaaatggt gcatttggtg aacaaggagt cgtcagaaac tccagaccag    2040
   tttatgacag ctgatgagac aaggaacctg cagaatgtgg acatgaagat tggggtg       2097
<210> 4
   <211> 699
   <212> PRT
   <213> HUMAN
<400> 4
   Met Asp Lys Phe Trp Trp His Ala Ala Trp Gly Leu Cys Leu Val Pro
   1               5                   10                  15
   Leu Ser Leu Ala Gln Ile Asp Leu Asn Ile Thr Cys Arg Phe Ala Gly
               20                  25                  30
   Val Phe His Val Glu Lys Asn Gly Arg Tyr Ser Ile Ser Arg Thr Glu
           35                  40                  45
   Ala Ala Asp Leu Cys Lys Ala Phe Asn Ser Thr Leu Pro Thr Met Ala
       50                  55                  60
   Gln Met Glu Lys Ala Leu Ser Ile Gly Phe Glu Thr Cys Arg Tyr Gly
   65                  70                  75                  80
   Phe Ile Glu Gly His Val Val Ile Pro Arg Ile His Pro Asn Ser Ile
                   85                  90                  95
   Cys Ala Ala Asn Asn Thr Gly Val Tyr Ile Leu Thr Ser Asn Thr Ser
               100                 105                 110
   Gln Tyr Asp Thr Tyr Cys Phe Asn Ala Ser Ala Pro Pro Glu Glu Asp
           115                 120                 125
   Cys Thr Ser Val Thr Asp Leu Pro Asn Ala Phe Asp Gly Pro Ile Thr
       130                 135                 140
   Ile Thr Ile Val Asn Arg Asp Gly Thr Arg Tyr Val Gln Lys Gly Glu
   145                 150                 155                 160
   Tyr Arg Thr Asn Pro Glu Asp Ile Tyr Pro Ser Asn Pro Thr Asp Asp
                   165                 170                 175
   Asp Val Ser Ser Gly Ser Ser Ser Glu Arg Ser Ser Thr Ser Gly Gly
               180                 185                 190
   Tyr Ile Phe Tyr Thr Phe Ser Thr Val His Pro Ile Pro Asp Glu Asp
           195                 200                 205
   Ser Pro Trp Ile Thr Asp Ser Thr Asp Arg Ile Pro Ala Thr Thr Thr
       210                 215                 220
   Ser Ser Asn Thr Ile Ser Ala Gly Trp Glu Pro Asn Glu Glu Asn Glu
   225                 230                 235                 240
   Asp Glu Arg Asp Arg His Leu Ser Phe Ser Gly Ser Gly Ile Asp Asp
                   245                 250                 255
   Asp Glu Asp Phe Ile Ser Ser Thr Ile Ser Thr Thr Pro Arg Ala Phe
               260                 265                 270
   Asp His Thr Lys Gln Asn Gln Asp Trp Thr Gln Trp Asn Pro Ser His
           275                 280                 285
   Ser Asn Pro Glu Val Leu Leu Gln Thr Thr Thr Arg Met Thr Asp Val
       290                 295                 300
   Asp Arg Asn Gly Thr Thr Ala Tyr Glu Gly Asn Trp Asn Pro Glu Ala
   305 310 315 320
   His Pro Pro Leu Ile His His Glu His His Glu Glu Glu Glu Thr Pro
   325 330 335
   His Ser Thr Ser Thr Ile Gln Ala Thr Pro Ser Ser Thr Thr Glu Glu
   340 345 350
   Thr Ala Thr Gln Lys Glu Gln Trp Phe Gly Asn Arg Trp His Glu Gly
   355 360 365
   Tyr Arg Gln Thr Pro Arg Glu Asp Ser His Ser Thr Thr Gly Thr Ala
   370 375 380
   Ala Ala Ser Ala His Thr Ser His Pro Met Gln Gly Arg Thr Thr Pro
   385 390 395 400
   Ser Pro Glu Asp Ser Ser Trp Thr Asp Phe Phe Asn Pro Ile Ser His
   405 410 415
   Pro Met Gly Arg Gly His Gln Ala Gly Arg Arg Met Asp Met Asp Ser
   420 425 430
   Ser His Ser Thr Thr Leu Gln Pro Thr Ala Asn Pro Asn Thr Gly Leu
   435 440 445
   Val Glu Asp Leu Asp Arg Thr Gly Pro Leu Ser Met Thr Thr Gln Gln
   450 455 460
   Ser Asn Ser Gln Ser Phe Ser Thr Ser His Glu Gly Leu Glu Glu Asp
   465 470 475 480
   Lys Asp His Pro Thr Thr Ser Thr Leu Thr Ser Ser Asn Arg Asn Asp
   485 490 495
   Val Thr Gly Gly Arg Arg Asp Pro Asn His Ser Glu Gly Ser Thr Thr
   500 505 510
   Leu Leu Glu Gly Tyr Thr Ser His Tyr Pro His Thr Lys Glu Ser Arg
   515 520 525
   Thr Phe Ile Pro Val Thr Ser Ala Lys Thr Gly Ser Phe Gly Val Thr
   530 535 540
   Ala Val Thr Val Gly Asp Ser Asn Ser Asn Val Asn Arg Ser Leu Ser
   545 550 555 560
   Gly Asp Gln Asp Thr Phe His Pro Ser Gly Gly Ser His Thr Thr His
   565 570 575
   Gly Ser Glu Ser Asp Gly His Ser His Gly Ser Gln Glu Gly Gly Ala
   580 585 590
   Asn Thr Thr Ser Gly Pro Ile Arg Thr Pro Gln Ile Pro Glu Trp Leu
   595 600 605
   Ile Ile Leu Ala Ser Leu Leu Ala Leu Ala Leu Ile Leu Ala Val Cys
   610 615 620
   Ile Ala Val Asn Ser Arg Arg Arg Cys Gly Gln Lys Lys Lys Leu Val
   625 630 635 640
   Ile Asn Ser Gly Asn Gly Ala Val Glu Asp Arg Lys Pro Ser Gly Leu
   645 650 655
   Asn Gly Glu Ala Ser Lys Ser Gln Glu Met Val His Leu Val Asn Lys
   660 665 670
   Glu Ser Ser Glu Thr Pro Asp Gln Phe Met Thr Ala Asp Glu Thr Arg
   675 680 685
   Asn Leu Gln Asn Val Asp Met Lys Ile Gly Val
   690 695
<210> 5
   <211> 1083
   <212> DNA
   <213> HUMAN
<400> 5
   atggacaagt tttggtggca cgcagcctgg ggactctgcc tcgtgccgct gagcctggcg 60
   cagatcgatt tgaatataac ctgccgcttt gcaggtgtat tccacgtgga gaaaaatggt 120
   cgctacagca tctctcggac ggaggccgct gacctctgca aggctttcaa tagcaccttg 180
   cccacaatgg cccagatgga gaaagctctg agcatcggat ttgagacctg caggtatggg 240
   ttcatagaag ggcacgtggt gattccccgg atccacccca actccatctg tgcagcaaac 300
   aacacagggg tgtacatcct cacatccaac acctcccagt atgacacata ttgcttcaat 360
   gcttcagctc cacctgaaga agattgtaca tcagtcacag acctgcccaa tgcctttgat 420
   ggaccaatta ccataactat tgttaaccgt gatggcaccc gctatgtcca gaaaggagaa 480
   tacagaacga atcctgaaga catctacccc agcaacccta ctgatgatga cgtgagcagc 540
   ggctcctcca gtgaaaggag cagcacttca ggaggttaca tcttttacac cttttctact 600
   gtacacccca tcccagacga agacagtccc tggatcaccg acagcacaga cagaatccct 660
   gctaccagag accaagacac attccacccc agtggggggt cccataccac tcatggatct 720
   gaatcagatg gacactcaca tgggagtcaa gaaggtggag caaacacaac ctctggtcct 780
   ataaggacac cccaaattcc agaatggctg atcatcttgg catccctctt ggccttggct 840
   ttgattcttg cagtttgcat tgcagtcaac agtcgaagaa ggtgtgggca gaagaaaaag 900
   ctagtgatca acagtggcaa tggagctgtg gaggacagaa agccaagtgg actcaacgga 960
   gaggccagca agtctcagga aatggtgcat ttggtgaaca aggagtcgtc agaaactcca 1020
   gaccagttta tgacagctga tgagacaagg aacctgcaga atgtggacat gaagattggg 1080
   gtg 1083
<210> 6
   <211> 361
   <212> PRT
   <213> HUMAN
<400> 6
   Met Asp Lys Phe Trp Trp His Ala Ala Trp Gly Leu Cys Leu Val Pro
   1 5 10 15
   Leu Ser Leu Ala Gln Ile Asp Leu Asn Ile Thr Cys Arg Phe Ala Gly
   20 25 30
   Val Phe His Val Glu Lys Asn Gly Arg Tyr Ser Ile Ser Arg Thr Glu
   35 40 45
   Ala Ala Asp Leu Cys Lys Ala Phe Asn Ser Thr Leu Pro Thr Met Ala
   50 55 60
   Gln Met Glu Lys Ala Leu Ser Ile Gly Phe Glu Thr Cys Arg Tyr Gly
   65 70 75 80
   Phe Ile Glu Gly His Val Val Ile Pro Arg Ile His Pro Asn Ser Ile
   85 90 95
   Cys Ala Ala Asn Asn Thr Gly Val Tyr Ile Leu Thr Ser Asn Thr Ser
   100 105 110
   Gln Tyr Asp Thr Tyr Cys Phe Asn Ala Ser Ala Pro Pro Glu Glu Asp
   115 120 125
   Cys Thr Ser Val Thr Asp Leu Pro Asn Ala Phe Asp Gly Pro Ile Thr
   130 135 140
   Ile Thr Ile Val Asn Arg Asp Gly Thr Arg Tyr Val Gln Lys Gly Glu
   145 150 155 160
   Tyr Arg Thr Asn Pro Glu Asp Ile Tyr Pro Ser Asn Pro Thr Asp Asp
   165 170 175
   Asp Val Ser Ser Gly Ser Ser Ser Glu Arg Ser Ser Thr Ser Gly Gly
               180                 185                 190
   Tyr Ile Phe Tyr Thr Phe Ser Thr Val His Pro Ile Pro Asp Glu Asp
           195                 200                 205
   Ser Pro Trp Ile Thr Asp Ser Thr Asp Arg Ile Pro Ala Thr Thr Asp
       210                 215                 220
   Gln Asp Thr Phe His Pro Ser Gly Gly Ser His Thr Thr His Gly Ser
   225                 230                 235                 240
   Glu Ser Asp Gly His Ser His Gly Ser Gln Glu Gly Gly Ala Asn Thr
                   245                 250                 255
   Thr Ser Gly Pro Ile Arg Thr Pro Gln Ile Pro Glu Trp Leu Ile Ile
               260                 265                 270
   Leu Ala Ser Leu Leu Ala Leu Ala Leu Ile Leu Ala Val Cys Ile Ala
           275                 280                 285
   Val Asn Ser Arg Arg Arg Cys Gly Gln Lys Lys Lys Leu Val Ile Asn
       290                 295                 300
   Ser Gly Asn Gly Ala Val Glu Asp Arg Lys Pro Ser Gly Leu Asn Gly
   305                 310                 315                 320
   Glu Ala Ser Lys Ser Gln Glu Met Val His Leu Val Asn Lys Glu Ser
                   325                 330                 335
   Ser Glu Thr Pro Asp Gln Phe Met Thr Ala Asp Glu Thr Arg Asn Leu
               340                 345                 350
   Gln Asn Val Asp Met Lys Ile Gly Val
           355                 360
<210> 7
   <211> 33
   <212> DNA
   <213> HUMAN
<400> 7
   gaattcgccg ccaccatgga caagttttgg tgg 33
<210> 8
   <211> 25
   <212> DNA
   <213> HUMAN
<400> 8
   tctagattac accccaatct tcatg 25
<210> 9
   <211> 35
   <212> DNA
   <213> HUMAN
<400> 9
   tatctagagc cgccaccatg gacaagtttt ggtgg 35
<210> 10
   <211> 30
   <212> DNA
   <213> HUMAN
<400> 10
   tatctagagc cattctggaa tttggggtgt 30
<210> 11
   <211> 22
   <212> DNA
   <213> HUMAN
<400> 11
   gatggagaaa gctctgagca tc 22
<210> 12
   <211> 19
   <212> DNA
   <213> HUMAN
<400> 12
   cgtcttcaaa taccatctc 19
<210> 13
   <211> 22
   <212> DNA
   <213> HUMAN
<400> 13
   tttgctccac cttcttgact cc 22
<210> 14
   <211> 30
   <212> PRT
   <213> HUMAN
<400> 14
   Ser Asn Pro Glu Val Leu Leu Gln Thr Thr Thr Arg Met Thr Ala Asp
   1               5                   10                  15
   Val Asp Arg Asn Gly Thr Thr Ala Tyr Glu Gly Asn Trp Asn
               20                  25                  30

## Description

The present invention relates to a hybridoma cell according to claim 1, a monoclonal antibody according to claim 2, an antibody molecule according to claim 3, a pharmaceutical composition according to claim 4, a method of purifying according to claim 6, a method for analyzing a sample according to claim 8, a method for identifying a tested individual according to claim 9, a method for identifying a tested individual according to claim 10, and the use of a pharmaceutical composition according to claim 13.

It is also disclosed antibodies specific for a CD44 variant protein derived from synovial cells of Rheumatoid Arthritis (RA) patients and to different biotechnological methods and uses concerning the same.

### LIST OF PRIOR ART

The following is a list of prior art, which is considered to be pertinent for describing the state of the art in the field of the invention. Acknowledgement of these references herein will be made by indicating within brackets the author's name and year of publication.
Golan I. et al., WO 0075312 (2000).
Aune, T.M., et al., Published EP Patent Application No. 501233 (1992).
Hale, L.P., et al., WO 9409811 (1994).
Herrlich *et al*., European Patent No. 538754, (1991).
Jalkanen, S., et al., WO 9500658, (1993).
Naor, D., et al., Adv. Cancer Res., 71:241, (1997).
Screaton, G.R., et al., Proc, Natl. Acad. Sci. USA, 89:12160, (1992).
Screaton et al. J. Biol. Chem. 268:12235 (1993).
Verdrengh, M., et al., Scand. J. Immunol., 42:353, (1995).
Nedvetzki et al., J. Autoimmunol., 13:39, (1999).

### BACKGROUND OF THE INVENTION

The cell surface adhesion molecule, designated CD44, has been shown to be implicated in cell-cell and cell-matrix interactions, as well as in cell traffic, cell transendothelial migration.

CD44 is a single chain molecule comprising a conserved amino terminal extracellular domain, a nonconserved membrane proximal region, a variable region expressing various combinations of variant exons, a conserved transmembrane spanning domain and a conserved cytoplasmic tail. The genomic sequence of CD44 includes 5 constant exons at the 5' terminus, and 5 constant exons at the 3' end. The mouse CD44 gene includes also 10 variant exons in the middle of the molecule, designated V₁-V₁₀, resulting in a total of 20 exons. The human CD44 gene comprises only 9 of these 10 variant exons (V₂-V₁₀) thus comprising a total of 19 exons (Screaton, G.R., *et al*., 1992). Differential V₂-V₁₀ alternative splicing generates many isoforms of CD44 that express various combinations of variant exons (designated exon Vx, x = 1-10), which are inserted in the membrane proximal domain and constitute the variable region of the molecule. These molecules are designated CD44 variants (CD44v). To date, a few dozen isoforms of CD44 are known.

In standard CD44 (CD44s, SEQ ID NO:5), constant exon number 5 is spliced directly to constant exon number 16 and therefore this molecule lacks the entire variable region. The resulting protein product is expressed predominantly on hematopoietic cells and therefore, this product is also known as hematopoietic CD44 (CD44H) or standard CD44 product (CD44s product, SEQ ID NO:6). In keratinocyte CD44, the longest CD44 identified so far, exon V3 to exon V10 are inserted in tandem between the two constant regions of the molecule.

The CD44 N-terminus contains the ligand binding site of the molecule. Hyaluronic acid (HA) is the principal ligand of CD44, but other extracellular matrix (ECM) components (e.g. laminin, collagen, fibronectin and chondroitin sulfate) as well as non-ECM constituents (mucosal vascular addressin, serglycin, osteopontin and class II invariant chain) can also interact with the CD44 receptor. Marked accumulation of CD44, and sometimes hyaluronic acid, is detected in areas of intensive cell migration and cell proliferation, as in wound healing, tissue remodeling, inflammation (including auto inflammation), morphogenesis and carcinogenesis.

The involvement of CD44 protein and variants thereof in autoimmune diseases is known. For example, it has been shown that anti-CD44 monoclonal antibodies (mAbs) can ameliorate the severity of experimentally induced autoimmune arthritis in mice (Verdrengh, M. *et al*. 1995). However, these mAbs are directed against the constant region of CD44 (and are thus designated anti-pan CD44 mAbs shared by all CD44 isoforms). Therefore, such mAbs may also block CD44 expressed on normal cells, which is required for migratory activity of immune and inflammatory cells engaged in microorganism eradication.

Monoclonal Abs directed against various variant regions of CD44 have also been suggested as potential agents for treatment of autoimmune diseases. Herrlich *et al.* describe mAbs directed against metastasis-specific variants of CD44v surface protein of a rat pancreatic adenocarcinoma (Herrlich *et al*., 1991). Anti-CD44-monoclonal antibodies, which inhibit T-cell proliferation, were also provided for treatment of various autoimmune diseases (Aune, T.M. *et al*., 1992). Monoclonal antibodies specific for forms of CD44 containing exon v6 were also reported as being useful for diagnosing inflammatory diseases (Jalkanen, S. et al., 1994). In addition, it has been reported (Hale, L.P., 1992) that administration of a CD44 protein, peptide or derivative can be used for treating various autoimmune diseases.

In an experimental arthritis mouse model (collagen-induced arthritis), injection of one of three different anti-CD44 mAbs, but not of the isotype-matched control mAbs, at disease onset, prevented an increase in footpad swelling and helped to maintain the clinical score at a very low level (Nedvetzki *et al*. 1999). Each of the three different types of anti-CD44 mAb recognized a distinct constant epitope of the CD44 receptor. All three antibodies displayed a similar anti-arthritic effect.

The involvement of CD44 in malignant processes has also been described (Naor, D., 1997). Anti-CD44 mAbs which were injected into mice, were shown to inhibit or prevent infiltration of various lymphoma and carcinoma cells into their target organs. In addition, transfection of a variant CD44 isoform into non metastatic rat pancreatic adenocarcinoma cells conferred metastatic potential to these cells.

### GLOSSARY

The following are terms which will be used throughout the description and claims and which should be understood in accordance with the invention to mean as follows:
***"Rheumatoid Arthritis-CD44 (CD44-RA) variant nucleic acid coding sequence"*** interchangeably referred to also as the **"*CD44vRA coding sequence"*** or ***"CD44-RA variant*"** - denotes nucleic acid molecules having the sequence shown in SEQ ID NO: 1. Nucleic acid molecules having at least 90% identity (see below) to said sequence and fragments (see below) of the above molecules of least 20 nucleotides long are likewise suitable for many of the same uses as the variant having SEQ ID NO:1. These molecules comprise sequences coding for a novel, naturally occurring, alternative splicing variant of the native and known CD44 transcript. It should be emphasized that the variant is a naturally occurring mature mRNA sequence resulting from alternative splicing of the primary mRNA transcript and not merely a truncated, mutated or fragmented form of the known sequence.

This CD44vRA sequence comprises exons 1-5, 15-17 and 19 of the constant part of the CD44 gene as well as exons 7-14 (v3-v10) of the variable region of the gene *(Screaton et al.* 1992, *supra*)*.* A characteristic feature of the variant coding sequence is that it comprises three additional bases (CAG) at the 5 end of Exon v5, in particular, in position 908-910 of SEQ ID NO:1, when optimally aligned with the wild type (CD44v3-v10) coding sequence, as expl**ained below.**

***"CD44vRA product"*** also referred to at times as *"****variant product",* "*CD44vRA protein*", *"variant protein", "CD44vRA peptide"*** or ***"variant peptide"*** is a polypeptide having an amino acid sequence encoded by the CD44vRA coding sequence. By ***"polypeptide"*** is intended a peptide or protein, as well as such having chemically modified amino acids (see below), e.g. a glycopeptide or glycoprotein. A characteristic feature of the CD44vRA product concerns the insertion, as compared to the wild type protein sequence (CD44v3-v10) when optimally aligned therewith, of a new Ala residue in position 303 of sequence ID NO:2, the latter being a preferred CD44vRA product according to the invention. This CD44vRA protein includes the native glycosylated form as isolated from human synovial cells of RA patients.

***"Nucleic acid molecule"*** or **"*Nucleic acid*"** denotes a single-stranded or double-stranded polymer composed of DNA nucleotides, RNA nucleotides or a combination of both types and may include natural nucleotides, chemically modified nucleotides and synthetic nucleotides.

**"*Amino acid sequence"*** a sequence composed of any one of the 20 naturally appearing amino acids, synthetic amino acids and amino acids which have been *chemically modified* (see below).

***"Antibody" -*** refers to antibodies of any of the classes IgG, IgM, IgD, IgA, and IgE antibody. The definition includes polyclonal antibodies or monoclonal antibodies. This term refers to whole antibodies or fragments of the antibodies, comprising the antigen-binding domain of the anti-variant product antibodies, e.g. scFv, Fab, F(ab')₂, other antibodies without the Fc portion, single chain antibodies, bispecific antibodies, diabodies, other fragments consisting of essentially only the variable, antigen-binding domain of the antibody, etc., which substantially retain the antigen-binding characteristics of the whole antibody from which they were derived.

The term **"*substantially retain the antigen binding characteristics of the whole antibody*"** should be understood to mean that the antibody fragment, derivative or the recombinant antibody molecule should be such that it specifically binds the CD44vRA product and that the affinity for the CD44vRA product as determined, for example, by Scatchard analysis (as described below) is at least 30% of the binding affinity of the whole antibody (from which the fragment, derivative or recombinant antibody molecule was derived). In preferred embodiments, the binding affinity of the antibody fragment, derivative or recombinant antibody molecule for the CD44vRA product is at least 50%, and more preferably is at least 75% of the binding affinity of the whole antibody.

***"Anti-CD44vRA antibody"*** or **"*Anti-CD44-RA variant antibody,"*** refers to an antibody that specifically binds to the CD44vRA protein. The antibody may include polyclonal or monoclonal antibodies, antibody fragments, antibody derivatives and homologues and recombinant antibody molecules all derived from the monoclonal or polyclonal anti-CD44vRA antibody. Anti-CD44vRA antibodies particularly include the monoclonal antibodies (mAbs) produced by the particular hybridoma described herein (referred to herein by the terms "*F8:33 mAb*" and "*F8:33-6-8-10 mAb*") as well as other antibodies that specifically bind to the CD44vRA protein (referred to as anti-CD44vRA mAbs). Preferably, the anti-CD44vRA antibody specifically binds to the epitope containing the additional Ala residue.

**"*Recombinant antibody molecule*"*-*** refers to an antibody molecule that results from manipulation of a monoclonal antibody, typically at the nucleic acid level (i.e., gene, mRNA, etc.), by standard genetic engineering techniques. The recombinant antibody molecule will be referred to as *"derived from*" the monoclonal antibody. Recombinant antibody molecules include, for example, chimeric, humanized, primatized, single chain antibodies and fusion proteins. The recombinant antibody molecule substantially retains the antigen binding characteristics of the monoclonal antibody from which it was derived.

**"*Antibody fragment*" *-*** includes a fragment of at least 6 amino acids of the anti-CD44vRA antibody or a fragment of at least 6 amino acids of a derivative or homologue of said polypeptide. The fragment will preferably comprise at least 10 amino acids, more preferably at least 20 amino acids, most preferably at least 30 amino acids, of said anti-CD44vRA antibody, or said derivative or homologue.

**"*Derivative of a monoclonal antibody (mAb)*" *-*** includes recombinant antibody molecules (as defined above) derived from the original mAb, as well as mAbs that are chemically modified (as defined above), and also includes mAb labeled with radioactive agents, fluorescent moieties, toxins, antibiotics, etc.

**"*Homologue of a monoclonal antibody (mAb)*"** - includes a protein having an amino acid sequence that is at least 90% identical to the sequence of the original mAb, or at least 90% identical to a fragment of at least 6 amino acids forming the original mAb. The variation in amino acid sequence between the homologue and the original mAb or a fragment thereof, arises from the addition, deletion, substitution or chemical modification of one or more amino acids of the original sequence. Where the homologue contains a substitution, the substitution is preferably a conservative one.

**"*Conservative substitution*"** - refers to the substitution of an amino acid in one class by an amino acid of the same class, where a class is defined by common physicochemical amino acid side chain properties and high substitution frequencies in homologous proteins found in nature, as determined, for example, by a standard Dayhoff frequency exchange matrix or BLOSUM matrix. Six general classes of amino acid side chains have been categorized and include: Class I (Cys); Class II (Ser, Thr, Pro, Ala, Gly); Class III (Asn, Asp, Gln, Glu); Class IV (His, Arg, Lys); Class V (Ile, Leu, Val, Met); and Class VI (Phe, Tyr, Trp). For example, substitution of an Asp for another class III residue such as Asn, Gln, or Glu is a conservative substitution.

***"Non-conservative substitution"** -* refers to the substitution of an amino acid in one class with an amino acid from another class; for example, substitution of an Ala, a class II residue, with a class III residue such as Asp, Asn, Glu, or Gln.

***"Chemically modified"** -* when referring to the product of the invention, means a product (protein, polypeptide, antibody) where at least one of its amino acid residues is modified either by natural processes, such as processing or other post-translational modifications, or by chemical modification techniques which are well known in the art. Among the numerous known modifications typical, but not exclusive examples include: acetylation, acylation, amidation, ADP-ribosylation, glycosylation, glycosaminoglycanation, GPI anchor formation, covalent attachment of a lipid or lipid derivative, methylation, myristlyation, pegylation, prenylation, phosphorylation, ubiqutination, or any similar process.

***"Optimal alignment" -*** is defined as an alignment giving the highest percent identity score. Such alignment can be performed using a variety of commercially available sequence analysis programs, such as the local alignment program LALIGN using a cutup of 1, default parameters and the default PAM.

***"Having at least 90% identity*"** - with respect to two sets of amino acid or nucleic acid sequences, refers to the percentage of residues that are identical in the two sequences when the sequences are optimally aligned. Thus, at least 90% amino acid sequence identity means that at least 90% of the amino acids in two or more optimally aligned polypeptide sequences are identical, however this definition explicitly excludes sequences which are 100% identical with the original mAb sequence.

*"Expression vector" -* refers to vectors that have the ability to incorporate and express DNA fragments in a cell. Many prokaryotic and eukaryotic expression vectors are known and/or commercially available. Selection of appropriate expression vectors is within the knowledge of those having skill in the art.

***"Deletion"** -* is a change in either nucleotide or amino acid sequence in which one or more nucleotides or amino acid residues, respectively, are absent as compared to the naturally occurring sequence.

***"Insertion"*** or ***"addition" -*** is that change in a nucleotide or amino acid sequence which has resulted in the addition of one or more nucleotides or amino acid residues, respectively, as compared to the naturally occurring sequence.

***"Substitution" -*** replacement of one or more nucleotides or amino acids by different nucleotides or amino acids, respectively as compared to the naturally occurring sequence. As regards amino acid sequences, the substitution may be conservative or non-conservative.

***"Biological sample"** -* The biological sample used in the methods of the invention can be any appropriate body-derived fluid sample including whole blood, peripheral blood monocytes, leukocytes, etc., preferably the biological sample will comprise synoviocytes or synovial fluid, or cellular extracts thereof.

***"Treating a disease"** -* refers to administering a therapeutic substance effective to ameliorate symptoms associated with a disease, disorder or pathological condition, in particular with Rheumatoid Arthritis (RA), to lessen the severity or cure the disease, or to prevent the disease from occurring, to prevent the manifestation of symptoms associated with the disease before they occur, to slow down the progression of the disease or the deterioration of the symptoms associated therewith, to enhance the onset of the remission period, to slow down the irreversible damage caused in the progressive chronic stage of the disease, to delay the onset of said progressive stage, to improve survival rate or more rapid recovery, or a combination of two or more of the above.

The treatment regimen will depend on the type of disease to be treated and may be determined by various considerations known to those skilled in the art of medicine, e.g. the physicians.

*"**Detection" -*** refers, in some aspects of the invention, to a method of detection of a disease, disorder, pathological or normal condition, and in particular, Rheumatoid arthritis and Psoriatic Arthritis. This term may refer to detection of a predisposition to the disease, disorder or pathological condition as well as for establishing the prognosis of the patient by determining the severity of the disease. Detection of the antibody-antigen complexes can be carried out by any of a number of techniques well known in the art, including, without limitation, those described in Harlow and Lane, Antibodies: A Laboratory Manual, Cold Spring Harbor Laboratory, 1988

WO 0075312 relates to a variant mRNA transcript of CD44 found in synovial cells of rheumatoid arthritis (RNA) patients is described. This transcript contains the known CD44 constant and variant exons but also comprises three additional nucleotides (CAG) that are transcribed from the end of the intron bridging Exon v4 to Exon v5 and are inserted at the 5' end of Exon v5. This extra CAG sequence results In the insertion of a new codon for the amino acid alanine. The CD44 transcript found to date in eighteen RA patients and not found in healthy (non-RA) individuals, is useful in the diagnosis, prognosis, prevention and treatment of RA, of other diseases in which the variant CD44 transcript is involved and possibly in disorders and diseases which involve cells expressing other forms of the CD44 protein.

Nedvetzki Shlomo et al, Journal of clinical investigation, vol. 111, N°8, April 2003, pages 1211-1220 discloses the CD44vRA variant specific for RA patients.

WO 03072606 is based on the finding that Gal-8 binds to carbohydrates of cell surface CD44std and CD44v and activates a chain of biological events within the cells. A specific effect exhibited was the induction of apoptosis in the CD44std and CD44v expressing cells. Thus, it concerns the use of an active agent for achieving a therapeutic effect on a target cell, the therapeutic effect comprises binding of said active agent to a standard CD44 (CD44std) glycoprotein or to a CD44 variant (CD44v) expressed by said target cell, the active agent being galectin-8 (Gal-8) or a functional derivative thereof. The active ingredient may be used for the preparation of a pharmaceutical composition, for the treatment of a disease or a disorder or for diagnostic purposes.

The hybridoma cell of the present invention is defined in claim 1, the monoclonal antibody is defined in claim 2, the antibody molecule is defined in claim 3, the pharmaceutical composition is defined in claim 4, the method of purifying is defined in claim 6, the method for analyzing a sample is defined in claim 8, the method for identifying a tested individual is defined in claim 9, the method for identifying a tested individual is defined in claim 10, and the use of a pharmaceutical composition is defined in claim 13.

The present invention is based on the development of hybridomas and monoclonal antibodies obtained therefrom, which specifically react with the CD44vRA product or with a fragment of said CD44vRA product, the fragment comprising an amino acid sequence translated from a region bridging exon v4 to exon v5 of CD44vRA coding sequence (SEQ ID NO:1) or from part of said region (e.g. 30 amino acids) and comprising an Ala residue which is not present in a corresponding fragment of CD44v3-v10 when CD44vRA product and CD44v3-v10 product (SEQ ID NO:4) are optimally aligned

The present invention also provides hybridoma cell-lines, designated herein *the F8:33 hybridoma* and *the F8:33-6-8-10 hybridoma* that produces monoclonal antibodies which specifically bind to CD44vRA product (SEQ ID NO:2). The hybridomas were deposited with the Collection Nationale De Cultures De Microorganismes (CNCM), Institut Pasteur, Paris, France, on April 16, 2003, deposit numbers: CNCM I-3015 (for hybridoma F8:33) and CNCM I-3016 (for hybridoma F8:33-6-8-10).

The invention further provides monoclonal antibodies (mAb) produced by either the F8:33 or the F8:33-6-8-10 hybridoma.

Fragments, derivatives or homologues of the above mAb also form part of the present invention, the fragments, derivatives or homologues substantially retaining the antigen binding specificity of the mAb of the invention.

In addition, the invention provides pharmaceutical compositions comprising the antibodies of the invention as defined above.

By another aspect, the invention provides a method of producing said antibodies, the method comprising (i) providing a cell of the F8:33 or F8:33-6-8-10 hybridoma that produces a Monoclonal antibody, and (ii) culturing said cell under conditions that permit production of said antibody.

By yet another aspect, the invention provides a method of isolating an antibody produced by the F8:33 or F8:33-6-8-10 hybridoma, comprising producing the antibody as described above, and isolating the antibody produced.

Further, the invention provides a method of purifying a polypeptide recognized by said antibody from a biological sample, the method comprising:
(i) providing an affinity matrix comprising said antibody bound to a solid support;
(ii) contacting said biological sample with the affinity matrix, to produce an antibody-polypeptide complex and an unbound biological sample;
(iii) removing the unbound biological sample; and
(iv) releasing the polypeptide from the antibody bound to the affinity matrix.

By yet a further aspect, the invention provides a method for identifying a tested individual having a high probability of having a disease, disorder or pathological condition involving cells which express an antigen having an epitope recognized by the antibody of the invention, the method comprises:
(i) obtaining a biological sample from said tested individual;
(ii) contacting the biological sample with said antibody under conditions enabling the formation of a detectable antibody-antigen complex; and
(iii) detecting said antibody-antigen complex, the presence of said antibody-antigen complex indicating a high probability that the tested individual has said disease or disorder.

In an alternative identification method according to the invention, the detection step (iii) comprises detecting said antibody-antigen complex and comparing the level of said complexes to the level of antibody-antigen complexes detected in samples obtained from a healthy individual, a deviation from said level indicating a high probability that the tested individual has said disease or disorder.

In a last aspect, the invention provides a method for achieving in an individual a therapeutic effect, comprising administering to said individual a therapeutically effective amount of the antibody of the invention, and obtaining a therapeutic effect in said individual, said effect being preventing or ameliorating the symptoms associated with the expression of an antigen comprising an epitope recognized by said antibody.

### BRIEF DESCRIPTION OF THE DRAWINGS

In order to understand the invention and to see how it may be carried out in practice, some non-limiting examples will now be described, with reference to the accompanying drawings, in which:

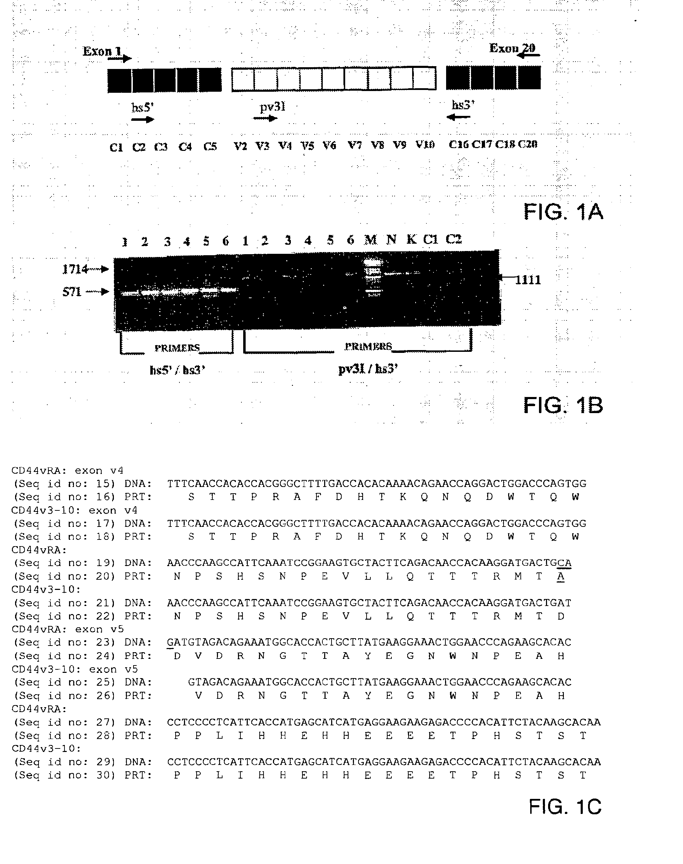

**Fig. 1A-1C** show the genomic structure of CD44 (Fig. 1A); agarose gel electrophoresis of primers representing the constant coding region of CD44 (Fig. 1B); and the partial nucleic acid sequence (exons 4 and 5) of RA-CD44 and CD44v3-v10, and the corresponding amino acid sequence (SEQ ID NO: 15-30), when the sequences are optionally aligned (Fig. 1C).

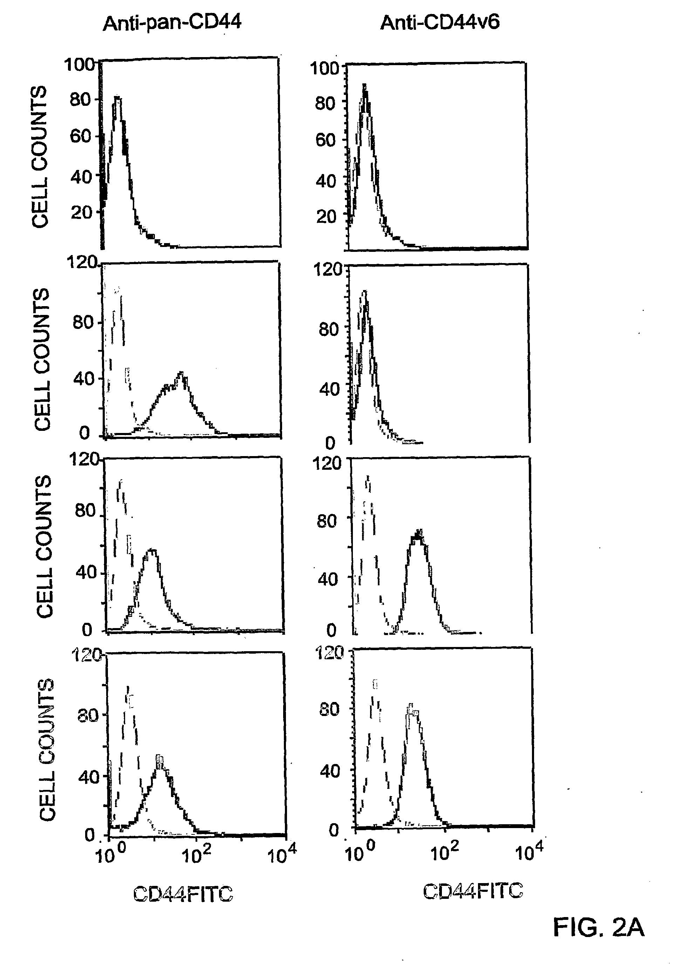

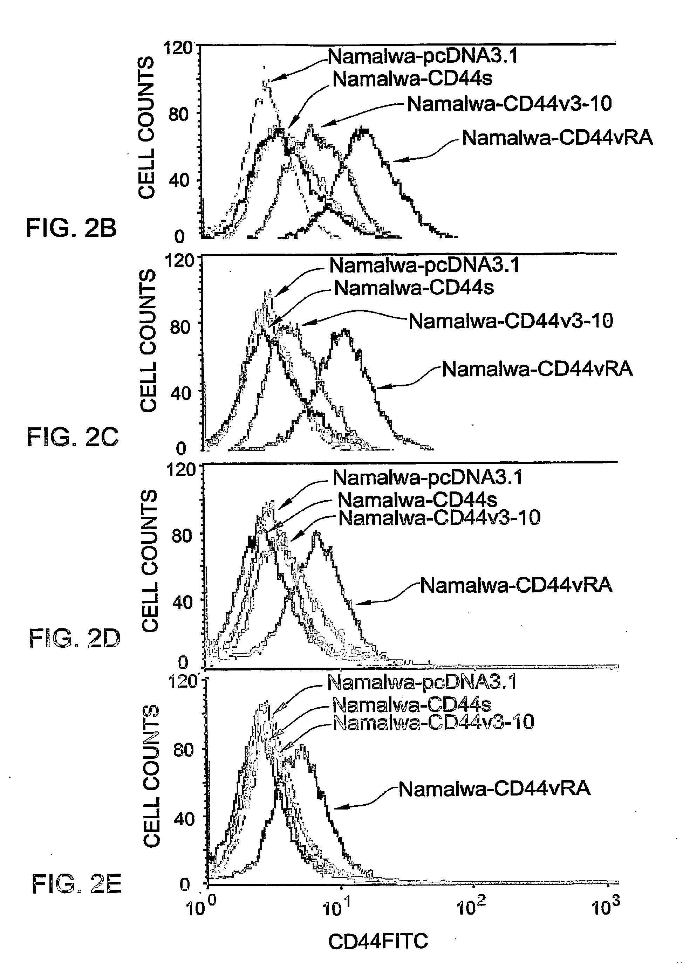

**Fig. 2A-2E** show histograms of fluorescence activated cell sorting (FACS) analysis of different Namalwa cells transfectants (as indicated), incubated with anti-pan-CD44 or anti- CD44v6 (Fig. 2A); the ability of mAb produced by F8:33 to bind, at 4µg/ml (Fig. 2B), 2µg/ml (Fig. 2C), 0.4µg/ml (Fig. 2D) and 0.2µg/ml (Fig. 2E), to the different Namalwa transfectants was also evaluated, wherein the binding of a fluorescein-coupled secondary antibody (IIAb) was used as the control. Figs. 2A-2E present cell count vs. mean fluorescence intensity of CD44FITC.

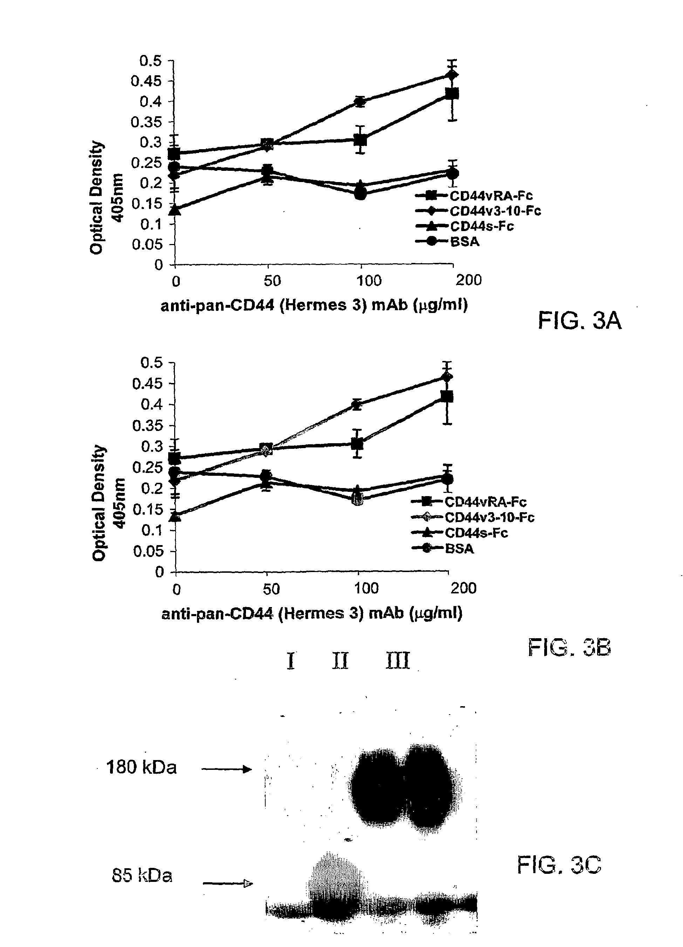

**Fig. 3A-3C** show the results of ELISA (enzyme-linked immunosorbent assay) analysis of F8:33 derived anti-CD44vRA mAb binding to microwells coated with soluble CD44vRA, CD44v3-v10, CD44s (CD44vRA-Fc (square), CD44v3-v10-Fc (rhombus), CD44s-Fc (triangle), respectively), while microwells with BSA (circle) served as the control (Fig. 3A). Binding of F8:33 derived anti-CD44 mAb was compared to that of anti-pan-CD44 mAb (Fig. 3B); Fig. 3C shows a Western Blot analysis of the different CD44 products (CD44s (lane II), CD44v3-v10 (lane II) and CD44vRA (lane III)).

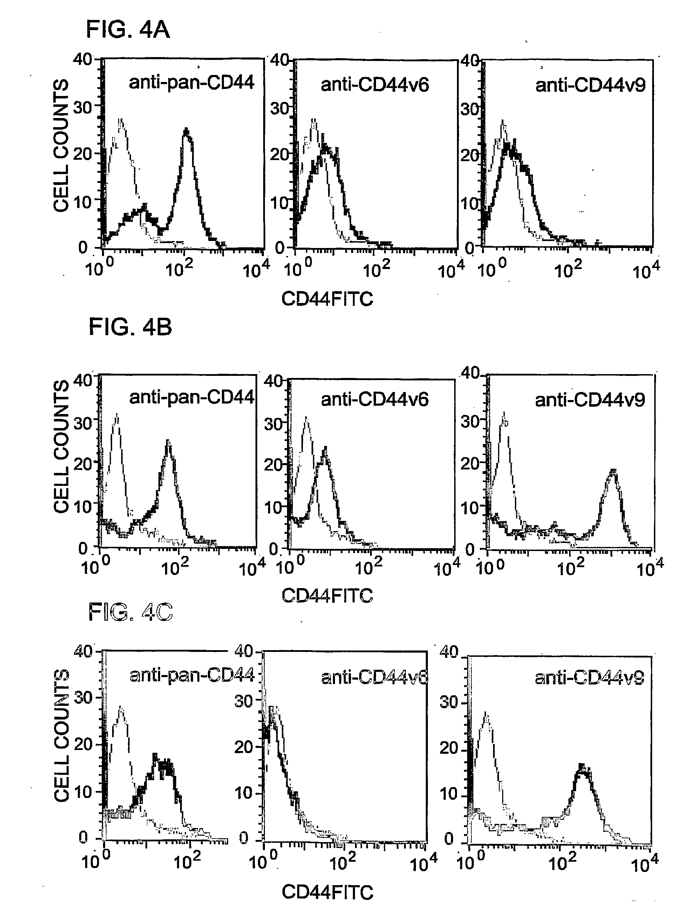

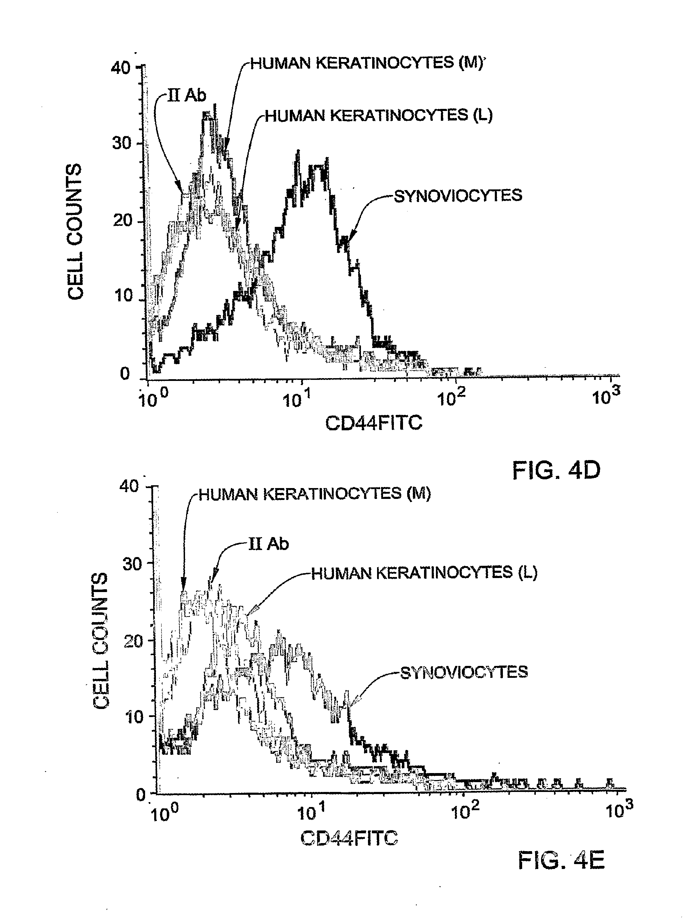

**Fig. 4A-4E** show FACS analysis of the binding of commercial anti-pan- from the joint of an RA patient (Fig. 4A) and to primary human keratinocytes provided from two individuals designated *donor M* (Fig, 4B) and *donor L* (Fig. 4C) as well as flow cytometry analysis of the selective binding of F8:33 derived anti-CD44vRA, at two concentrations, 4µg/ml (Fig. 4D) and 2µg/ml (Fig. 4E) to primary keratinocytes from the same two individuals and to synoviocytes. Figs. 4A-4E present cell count vs. mean fluorescence intensity of CD44FITC.

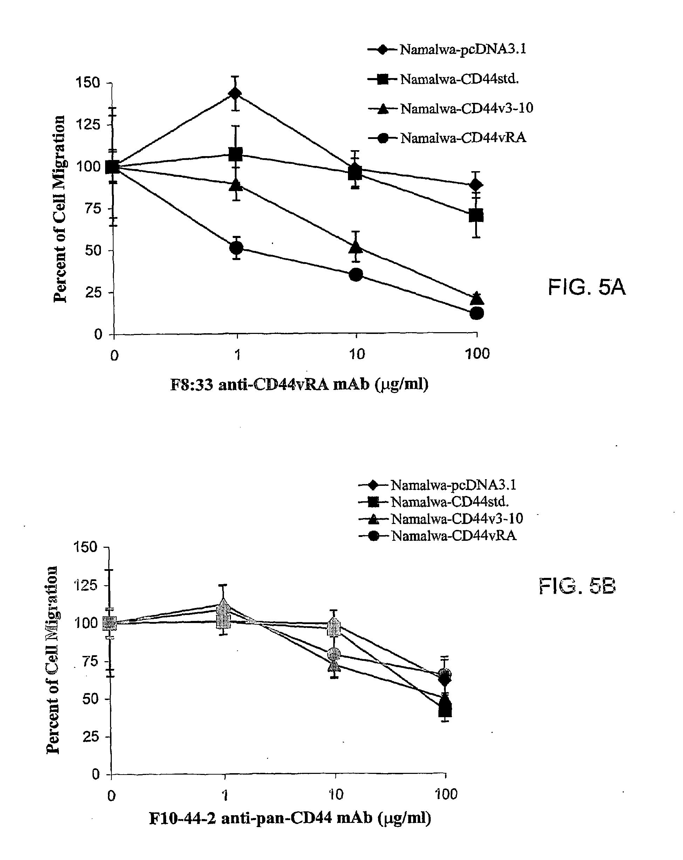

**Fig. 5A-5B** show graphs of cell migration assays performed in the presence or absence of F8:33 anti-CD44vRA mAb (Fig. 5A) or of anti-pan CD44 mAb and according to which Namalwa transfectants (Namalwa-pcDNA3.1 rhombus, Namalwa-CD44s square, Namalwa-CD44v3-v10 triangle, and Namalwa-CD44vRA circle) were analyzed, in the absence or the presence of F8:33 anti-CD44vRA mAb (Fig. 5A) or anti-pan CD44 mAb (Fig. 5B) for their ability to cross HA-coated filters in a transwell migration assay and the percentage of cell migration was calculated by the number of cells that crossed the membrane in the presence of antibody, divided by the number of cells that crossed the membrane in its absence, x 100.

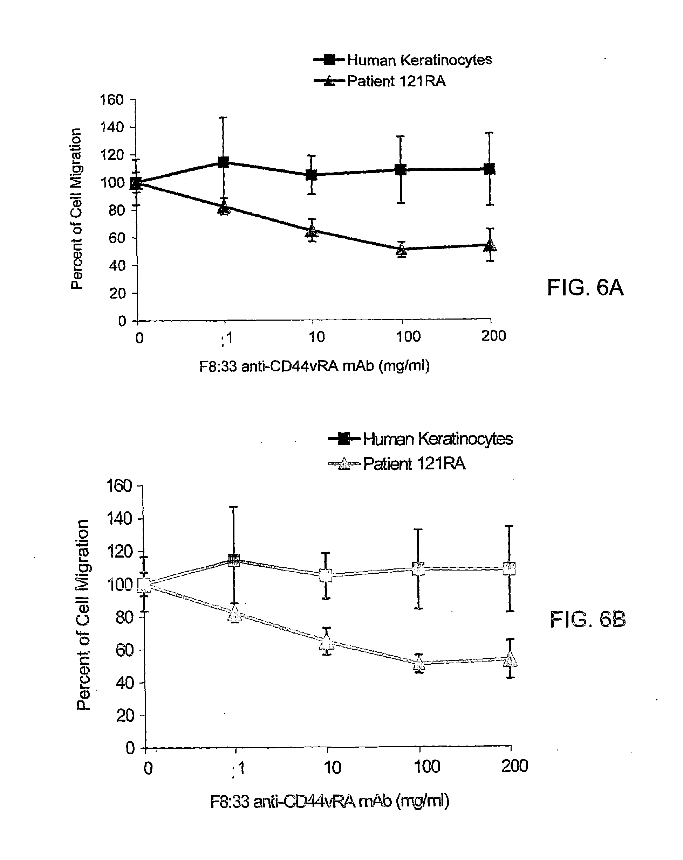

**Fig. 6A-6B** show graphs of cell migration assays performed in the presence or absence of F8:33 derived anti-CD44vRA mAb (Fig. 6A) or of anti-pan CD44 mAb (Fig. 6B) using synovial fluid cells from a joint of an RA patient (triangle), or primary keratinocytes (square), and evaluating their ability to cross HA-coated filters in a transwell migration assay. (The percentage of cell migration was calculated as in Fig. 5B).

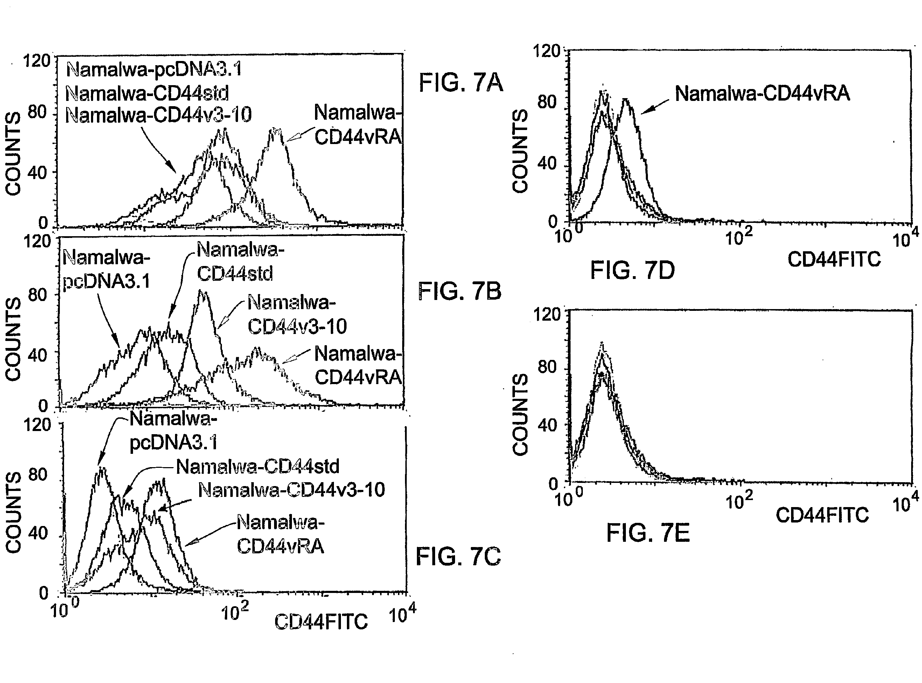

**Fig. 7A-7E** show FACS analysis of the binding of F8:33-6-8-10 derived anti-CD44-vRA mAbs to Namalwa-pcDNA3.1 cells, Namalwa-CD44s cells, Namalwa-CD44v3-v10 cells or Namalwa-CD44vRA cells (cell count vs. mean fluorescence intensity of CD44FITC). The antibody was present in the following concentrations: 1.2 mg/ml (Fig. 7A), 120 µg/ml (Fig. 7B), 12 µg/ml (Fig. 7C), 1.2 µg/ml (Fig. 7D) and 120 ng/ml (Fig. 7E).

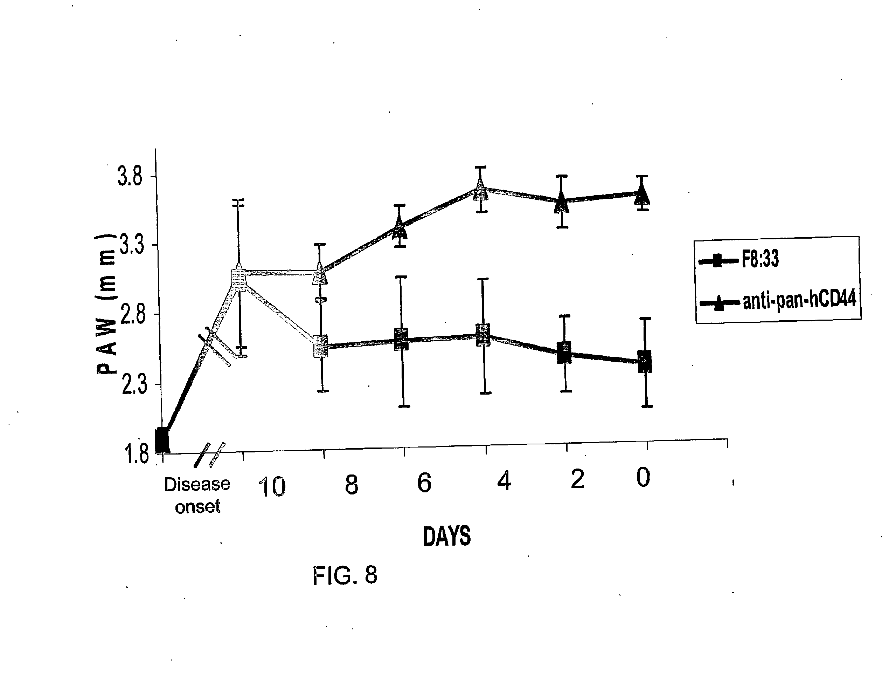

**Fig. 8** shows the effect of F8:33 (squares) on Collagen-Induced Arthritis (CIA) in mice. Arthritis development was monitored for 10 days by measuring paw swelling. The values are the mean ± SEM. Anti-pan-hCD44 was used as control (triangles).

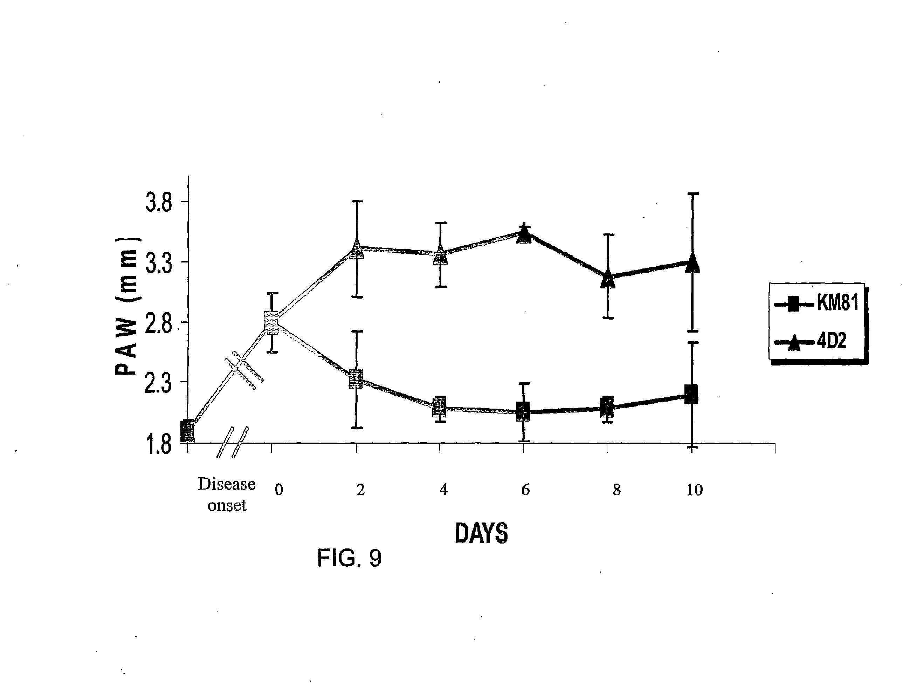

**Fig. 9** shows the effect of KM81 (anti-pan mouse-CD44, squares) and 4D2 (control isotype-matched antibody, triangles) on CIA in mice. The values are the mean ± SEM.

### DETAILED DESCRIPTION OF THE INVENTION

In accordance with the present invention, two hybridoma cell lines have now been isolated that produce monoclonal antibodies (mAbs). These mAbs were found to specifically bind to the CD44vRA product (CD44vRA) present in synovial cells of individuals having rheumatoid arthritis (RA) but not present in the synovial cells of non-RA individuals. Because the mAbs are specific for the CD44vRA product in preference to the original, wild type protein sequence (CD44v3-v10) or other isoforms of CD44, the monoclonal antibodies are useful, *inter alia,* in a variety of methods requiring the identification, isolation or targeting of the CD44vRA product.

The present invention thus concerns, according to a first of its aspects, hybridomas and monoclonal antibodies expressed thereby, which react with specificity to CD44vRA product or to a fragment of said CD44vRA product, the fragment comprising an amino acid sequence translated from a region bridging exon v4 to exon v5 of CD44vRA coding sequence or from part of said region and comprising at least the Ala residue which is not present in a corresponding fragment of the wild type variant, CD44v3-v10, when CD44vRA and CD44v3-v10 are optimally aligned. This Ala residue corresponds to the Ala residue in position 303 of the variant product CD44vRA, the sequence of which is depicted in SEQ ID NO:2.

The invention also concerns mouse hybridoma cell lines having the depository Accession Nos. CNCM I-3015 (for hybridoma F8:33) and CNCM I-3016 (for hybridoma F8:33-6-8-10), (referred to herein as the *F8:33* and *F8:33-6-8-10* hybridoma cell lines), deposited with the Collection National De Cultures De Microorganisms (CNCM), Institut Pasteur, Paris, France, on April 16, 2003) and to mAbs produced by said cell line, clones and subclones. The hybridomas may be produced by any of the methods known in the art [e.g. as described by Kohler, G., and Milstein C. in Nature 256:495-497 (1975)]. The supernatants of the hybridoma cells are typically screened for antibody binding activity by any one of the methods known in the art such as by enzyme linked immunosorbent assay (ELISA) or radio-immunoassay (RIA). The supernatants can be screened for production of mAbs capable of binding to any of the CD44vRA products (including fragments, derivatives or homologues thereof) or to cells expressing said products.

The monoclonal antibodies are typically produced by culturing the hybridoma cells under conditions suitable to produce the monoclonal antibody and isolating the mAb from the cell culture by well known techniques. Such conditions and techniques are well known in the art and are described in Mammalian cell biotechnology: a practical approach (Butler, M. Ed, IRL Press). The anti-CD44vRA antibodies of the invention may also be produced by recombinant genetic methods well known to a person skilled in the art; for example, as described in DNA Cloning 4: a practical approach, Chapter 3 (Glover, D. and Hames, B. Eds. IRL Press) and Bebbington et al. [Bio/Technology 10:169 (1992)].

As indicated hereinbefore, the recombinant antibody molecules include, for example, chimeric antibodies [as described by Morrison S.L, Science 229:1202 (1985)], humanized antibodies [as described by, for example, Shin S.U. and Morrison S.L., Methods Enzymol., 178:459-476, (1989); Gussow D. and Seemann G., Methods Enzymol. 203:99-121 (1991)], bispecific. antibodies [as described by, for example, Weiner L.M. et al., J. Immunol. 151:2877-2886 (1993); Goodwin D.A., Int. J. Rad. Appl. Instrum. 16:645-65 (1989)], single chain antibodies (scFv, as described by, for example, gritzapis A.D., et al. Br. J. Cancer 88:1292-1300, (2003)]), complete or fragmentary immunoglobulins [as described by, for example, Coloma M.J., et al., J. Immunol. Methods, 152:89:104, (1992); Nesbit M., et al., J. Immunol. Methods, 151:201-208 (1992); Barbas C.F., et al., Proc. Natl. Acad. Sci. USA, 89:10164:10168, (1992)], or antibodies generated by chain shuffling [as described by, for example, Winter G., et al., Annu. Rev. Immunol., 12:433-455, (1994)]. Humanized antibodies may be produced, for example, by CDR grafting (e.g. as described in published European patent application No. 0239400). Framework regions may also be modified (e.g. as described in European patent application No. 0519596). To humanize antibodies, methods such as PCR (for example, as described in European patent application Nos. 0368684; 0438310; or in international patent publication No. WO 92/07075) or computer modeling (for example, as described in international patent publication No. WO 92/22653) may be used. Fusion proteins, e.g. single chain antibody/toxin fusion proteins [as described, for example, by Chaudhary VK., et al., Proc. Natl. Acad. Sci. USA, 87:9491-9494 (1990); Friedman P.N. et al., Cancer Res. 53:334-339, (1993)] may also be produced and thus also form part of the present invention.

The hybridoma cell line of the present invention comprises a nucleic acid encoding the monoclonal antibodies and such nucleic acid is also within the scope of the present invention. Nucleic acid encoding the anti-CD44vRA antibodies can be isolated from hybridoma cell lines by techniques that are well known in the art including those described in Antibody engineering: a practical approach McCafferty et al., Eds. IRL Press). Such nucleic acids are useful for preparation of additional cell lines or transgenic animals expressing the anti-CD44vRA antibodies or may be used in the preparation of recombinant antibody molecules.

The anti-CD44vRA Abs of the present invention include the monoclonal antibodies expressed by the hybridoma cell line of the present invention, whether actually produced in the hybridoma cells or produced by other techniques as are well known in the art; for example, produced in other cell types by transfer of the appropriate genetic material from the hybridoma cell (see for example Monoclonal antibodies: the second generation. Zola, H. Ed. BIOS Scientific, Chapters 4-9). Accordingly, there is also provided a monoclonal antibody as defined above, i.e. that reacts, similarly to the F8:33 or F8:33-6-8-10 derived mAbs, with specificity to CD44vRA or to a fragment of said CD44vRA, the CD44vRA fragment comprising an amino acid sequence translated from the region flanking exon v4 to exon v5 of CD44vRA coding sequence or from part of said region and comprising an Ala residue (corresponding to the Ala residue in position 303 in SEQ ID NO : 2), which is not present in a corresponding fragment of CD44v3-vlO when CD44vRA and CD44v3-v10 are optimally aligned.

The CD44vRA mRNA transcript contains the constant exons 1-5, 15-17 and 19, and variant exons 7-14 (v3-v10) [as described by Screaton et al. (1992) *ibid.*] and also the three additional nucleotides in position 908-910 of SEQ ID NO:1, which result in the insertion of the new Ala residue in position 303 of SEQ ID NO:2. The generation of the extra CAG in the CD44 transcript of RA patients' synoviocytes is shown in Fig. 1C. The CAG inclusion in the CD44 mRNA allows insertion of the Ala residue into the translated cell surface CD44 glycoprotein. This change is sufficient to allow the production of the F8:33 or F8:33-6-8-10 mAbs which recognize CD44vRA expressed on synovial fluid cells of RA patients.

The illegitimate transcription of the intronic CAG flanking exon v5 of CD44vRA is presumably a consequence of mis-regulation of the splicing machinery in this molecule. Unknown genetic or environmental factors or a combination of both may modify the relative abundance, tissue distribution or activity of serine-arginine (SR) or of heterogeneous nuclear ribonucleoprotein (hnRNP) splicing factors that antagonistically control differential splicing [Caceres, J.R, and Kombliht, A.R. Trends Genet. 18:186-193 (2002)]. The CAG-containing splice junction may be particularly susceptible to such changes, resulting in the CAG inclusion in the CD44 mRNA sequence of RA patients. A similar mechanism may influence the CD44 splicing machinery in other autoimmune diseases. In this context, it should be mentioned that mutations located in non-coding regions such as those affecting 5' and 3' splice sites, branch sites or polyadenylation signals, are frequently (~15%) the cause of genetic diseases [Krawczak, M., Reiss, J., and Cooper, D.N. Hum. Genet. 90: 41-54 (1992)].

In view of the fact that the CD44vRA product contains a unique additional region which does not appear in CD44 obtained from synoviocytes from healthy individuals, antibodies *specifically directed against* CD44vRA product can thus be used for diagnosis, prognosis, prevention and therapy of various diseases in which cells expressing the CD44 molecule are involved.

By ***"specifically directed against"*** or ***"specifically bind"*** to the CD44vRA product is meant that the antibody recognizes and binds to the CD44vRA product in preference to other known CD44 proteins; in particular, the anti-CD44vRA antibodies recognize and bind to the CD44vRA product in preference to the original, wild type, protein sequence, designated CD44v3-v10. Generally, the affinity of anti-CD44vRA antibodies is at least 2-fold greater for binding to the CD44vRA than for binding to the original, wild type, protein sequence or other CD44 isoforms. In a preferred embodiment, the binding affinity of anti-CD44vRA antibodies is at least 10-fold greater for binding to the CD44vRA than for binding to the original, wild type, CD44v3-v10 protein sequence or other CD44 isoforms. The binding affinity may be determined by a Scatchard analysis on antigens present on cell surfaces, a method that is well known in the art (see, for example, Hulmes, E.C. in Receptor-ligand Interactions: a practical approach Chapter 4. Rickwood and Hames, Eds. IRL Press.).

Specific binding may also be inferred from biological assays, such as the effect of the antibody on cell migration, therapeutic effects, the ability to induce cell aggregation, immunofluorescent studies, binding-competition assays and the like. In such assays, an antibody would be defined as CD44vRA specific if the results using this antibody in the experimental setting differ in a statistically significant manner from those of the control settings.

The addition of the single Ala amino acid in position 303 in the CD44vRA product resulted in the formation of an antigenically distinct epitope in the CD44vRA product. Thus, the present invention also concerns homologues, fragments and derivatives (such as chemically modified derivatives, radiolabeled derivatives, derivatives coupled to toxin or antibiotic molecules, and the like) of the antibodies as defined, all recognizing the antigenically distinct epitope (that includes the additional Ala) and thus substantially retaining the antigen binding specificity of the monoclonal antibodies.

Also, recombinant antibody molecules that are derived from the monoclonal antibodies produced by the hybridoma cell lines F8:33 and F8:33-6-8-10 as well as additional hybridomas producing anti-CD44vRA mAbs and their clones and subclones that substantially retain the antigen binding characteristics of the monoclonal antibody are explicitly included in the present invention. It is within the skills of the average artisan to prepare homologues, fragments and derivatives of the antibody of the invention or, starting from a sequence analysis of the antibody and/or by use of the hybridoma cell line producing this antibody, to prepare recombinant antibody molecules with the same idiotype, i.e. antibody molecules having the same amino acid sequence in the region of the antigen binding site (complementarity-determining regions, CDR) as the antibody from hybridoma cell lines F8:33 and F8:33-6-8-10 or other hybridomas producing mAbs recognizing CD44vRA and their clones and subclones.

Fragments of the antibodies that fall under the scope of the present invention may be, for example, Fab, F(ab')₂, Fv or scFv fragment or any other fragments of the monoclonal antibody molecule that substantially retain the antigen binding characteristics of the whole antibody. Fragments can be produced by chemical or enzymatic cleavage of the whole antibody or by any of the methods that are well known in the art. Fragments can also be produced by recombinant methods that are well known in the art, e.g., by expressing portions of the antibody genes (heavy or light chain or both) in a heterologous host.

As indicated above, the anti-CD44vRA antibody of the present invention will recognize an epitope of the CD44vRA product that is not present in the original, wild type protein sequence (CD44v3-vlO). The anti-CD44vRA antibodies of the invention preferably recognize an epitope comprising the Ala at residue 303 of SEQIDNO:2. Alternatively, or in addition, the anti-CD44vRA antibodies of the invention recognize a neo-epitope created by a change in the overall tertiary structure of the CD44 protein as a consequence of the Ala residue insertion in position 303. Such neo-epitopes can be conformational epitopes, nonlinear epitopes, carbohydrate epitopes or epitopes exposed by differential glycosylation. Epitopes may include the variant region of the peptide sequence at the beginning of the v5 exon or the epitope may be on a different part of the molecule whose tertiary structure is altered by the insertion of the Ala residue of the variant polypeptide.

Various hosts can be used for production of antibodies by the hybridoma technique including rats, mice, etc. The animals may be immunized by injecting the host with the CD44vRA product (including said fragments, derivatives or homologues). Various adjuvants may be used to increase the immunological response to the antibodies, such as Freund's, mineral gels, aluminum hydroxide, etc. The animal hosts may also be immunized with cells, for example human Namalwa cells, that have been transfected with vectors carrying genetic material encoding the CD44vRA products (including fragments, derivatives or homologues thereof) as described herein.

In addition to the hybridoma technique mentioned above, clones and subclones of this hybridoma as well as continuous cell lines which produce antibodies obtained by additional techniques may also be used such as, for example, the EBV-hybridoma technique [Cole et al., Mol. Cell Biol. 62:109 (1984)].

In accordance with a therapeutic aspect the invention provides pharmaceutical compositions comprising a pharmaceutically acceptable carrier and, as an active ingredient, an amount of the monoclonal anti-CD44vRA antibodies of the invention, including homologues, fragments or derivatives thereof and recombinant antibody molecules derived from the same, all of which substantially retain the antigen binding specificity of the original monoclonal antibodies, the amount being effective to achieve a therapeutic effect, e.g. on the immune system of a subject in need of such treatment, as further discussed below.

The mAb of the present invention is administered and dosed in accordance with good medical practice, taking into account the clinical condition of the individual patient, the site and method of administration, scheduling of administration, patient age, sex, body weight and other factors known to medical practitioners.

The "***amount***" for purposes herein is determined by such considerations as known in the art. The amount must be effective to achieve the desired therapeutic effect as described above, e.g. neutralization of the CD44vRA product or blocking its binding to its target, for example, by the neutralizing effect of the antibodies. The amount depends, *inter alia,* on the type and severity of the disease to be treated and the treatment regime. The amount is typically determined in appropriately designed clinical trials (dose range studies) and the person versed in the art will know how to properly conduct such trials in order to determine the effective amount. As generally known, an effective amount depends on a variety of factors including the affinity of the antibody to the cell surface CD44vRA glycoprotein, its distribution profile within the body, a variety of pharmacological parameters such as half life in the body, undesired side effects, if any, factors such as age and gender of the treated individual, etc.

By the term **"*pharmaceutically acceptable carrier***" it is meant any inert, non-toxic material, which does not react with the mAb of the invention. Thus, the carrier can be any of those conventionally used and is limited only by chemico- physical considerations, such as solubility and lack of reactivity with the compound, and by the route of administration.

The carriers may also refer to substances added to pharmaceutical compositions to give a form or consistency to the composition when given in a specific form, e.g. in a form suitable for injection, in pill form, as a simple syrup, aromatic powder etc. The carriers may also be substances for providing the composition with stability (e.g. preservatives) or for providing the formulation with an edible flavor.

The choice of carrier will be determined in part by the particular mAb, as well as by the particular method used to administer the composition. Accordingly, there is a wide variety of suitable formulations of the pharmaceutical composition of the present invention. A preferred formulation is that suitable for parenteral administration, for example subcutaneous, intravenous, intraperitoneal or intramuscular, either systemically or locally. The requirements for effective pharmaceutical carriers for injectable compositions are well known to those of ordinary skill in the art. See, for example, Pharmaceutics and Pharmacy Practice, J.B. Lippincott Co., Philadelphia, Pa., Banker and Chalmers, eds., pages 238-250 (1982), and ASHP Handbook on Injectable Drugs, Toissel, 4th ed., pages 622-630 (1986). It may also be administered by intravenous infusion.

As an example, for the preparation of a pharmaceutical composition suitable for parenteral administration, e.g. intravenously by *iv drip* or *infusion,* dosages in the range of from 1 mg to 10 mg per kg body weight (the lower concentrations are preferable). However, the pharmaceutical composition for treatment in the method of the invention is not limited to these dosages, and other appropriate dosages are well within the competence of the skilled artisan to select.

Carriers suitable for injectable formulations of the compositions of the invention may include, without being limited thereto, vegetable oils, dimethylacetamide, dimethylformamide, ethyl lactate, ethyl carbonate, isopropyl myristate, ethanol, polyols (glycerol, propylene glycol, liquid polyethylene glycol, and the like). For intravenous injections, water-soluble versions of the therapeutic agent may be administered by the drip method, whereby a pharmaceutical formulation containing antibody and a pharmaceutically acceptable carrier is infused. Specific pharmaceutically acceptable carriers may include, for example, 5% dextrose, 0.9% saline, Ringer's solution or other suitable excipients. Intramuscular preparations, e.g., a sterile formulation of a suitable soluble salt form of the antibody, can be dissolved and administered in a pharmaceutical excipient such as Water-for-Injection, 0.9% saline, or 5% glucose solution. A suitable insoluble form of the antibody mary be prepared and administered as a suspension in an aqueous base or a pharmaceutically acceptable oil base, such as an ester of a long chain fatty acid (e.g., ethyl oleate). As should be appreciated, the pharmaceutical composition may be in the form of a medical formulation kit, together with at least one type of medical carrier or diluent. The antibodies may be conjugated to radioactive, enzymatic, antibiotic or toxic agents.

The pharmaceutical compositions of the invention are suitable for the prevention or treatment of diseases, disorders or pathological conditions where a therapeutically beneficial effect may be achieved by neutralizing the CD44vRA protein or blocking its binding to its target, for example, by the neutralizing effect of the antibodies. Such diseases, disorders or pathological conditions may, for example, be inflammatory diseases or inflammatory complications of infectious diseases, autoimmune diseases, malignant diseases or any other disease or disorder in which cells comprising the CD44vRA or expressing the CD44vRA protein are involved. Autoimmune diseases include rheumatoid arthritis, systemic lupus erythematosus, insulin-dependent diabetes, multiple sclerosis, and inflammatory bowel diseases (including Crohn's disease and ulcerative colitis). In addition, such pharmaceutical compositions may be used for the treatment of diseases which involve cells expressing other forms of the CD44 protein that include the unique Ala-containing epitope.

In accordance with a preferred embodiment, the pharmaceutical composition comprising said anti-CD44vRA antibodies is used for the treatment of autoimmune diseases.

In accordance with the preparative aspect of the invention, a method of making an antibody as defined above is provided, the method comprising:
(i) providing a cell of the F8:33 or F8:33-6-8-10 hybridomas or other hybridomas producing anti-Cd44vRA mAbs or their clones or subclones;
(ii) culturing said cell under conditions that permit production of antibodies.

In the specific embodiment of the invention, the hybridoma cells grew in RPMI-1640 medium (cat. no. R-8758, Sigma ltd., Israel) including 10% of fetal calf serum (cat. no. CH30160.03, Perbio, Belgium) and 1% of pen-strep antibiotics (cat. no. 03-031-1B, Bet-Haemek ltd., Israel). After growing of 70%-80% culture confluents the cells were transferred to low protein medium (LPM, cat. no. 05-040-1A, Bet-Haemek ltd., Israel) for one week.

The preparative method of the invention may also include the step of isolating the antibody thereby produced. According to the specific embodiment of the invention, the purification of the antibody was performed using HiTrap^{™} Protein G HP column (cat. no. 17-0405-01, Amersham Biosciences AB, Sweden) according of manufacturing instructions.

Yet, within this aspect, there is provided a method of purifying from a biological sample a polypeptide or protein containing an epitope recognized by the antibody of the invention, the method comprising:
(i) providing an affinity matrix comprising said antibody bound to a solid support;
(ii) contacting said biological sample with the affinity matrix, to produce an antibody-polypeptide complex and unbound biological sample;
(iii) removing the unbound biological sample; and
(iv) releasing the polypeptide from the antibody bound to the affinity matrix.

The polypeptide is preferably the CD44vRA product.

The complex can be separated from the remainder of the sample by any number of standard techniques, for example, by physically removing the complex from the sample or by washing the complex. The bound CD44vRA product may then be released from the complex to provide CD44vRA product that is substantially purified. Solid supports suitable for this method are well known in the art, for example, agarose, polyacrylamide or polyacrylic beads, including magnetic and protein A coupled beads.

In accordance with a diagnostic aspect of the invention, methods for identifying an individual having a high probability of having a disease, disorder or pathological condition involving cells which express the RA-CD44 variant protein is also contemplated.

A variety of protocols useful for assaying the CD44vRA product, using either polyclonal or monoclonal antibodies, are known in the art. Examples include enzyme-linked immunosorbent assay (ELISA), radio-immunoassay (RIA), and fluorescence activated cell sorting (FACS). Direct or competitive binding assays may also be used. These diagnostic assays utilize the anti-CD44vRA antibody and a label to detect CD44vRA product in human body fluids or extracts of cells or tissues. The antibodies of the present invention may be used with or without modification. Frequently, the antibodies will be labeled by joining them, either covalently or non-covalently, with a reporter molecule. Such a reporter molecule may for example be a radioactive agent, a fluorescent agent (e.g. FITC), an enzyme, etc.

A diagnostic method according to the invention comprises:
(i) obtaining a biological sample from a tested individual;
(ii) contacting said biological sample with an antibody, as defmed herein, under conditions enabling the formation of a detectable antibody-antigen complex; and
(iii) detecting said antibody-antigen complex, the presence of said antibody-antigen complex indicating a high probability that the tested individual has a disease or disorder involving cells which express the CD44vRA protein.

Alternatively, the diagnostic method of the invention may comprises:
(i) obtaining a biological sample from a tested individual;
(ii) contacting said biological sample with an antibody, as defined herein, under conditions enabling the formation of a detectable antibody-antigen complex; and
(iii) detecting said antibody-antigen complex and comparing the level of said complexes to the level of antibody-antigen complexes detected in a sample obtained from a healthy individual, a deviation from said level indicating a high probability that the tested individual has said disease or disorder.

In one particular embodiment, the monoclonal antibodies, fragments and derivatives thereof are useful in a method for identifying an individual having a high probability of having Rheumatoid Arthritis (RA).

The above diagnostic methods may be quantified in ways that are known in the art to determine the level or amount of a protein in a sample (in this particular case, the level of CD44vRA product), which may be indicative of the kind or extent of the disease that the individual has. The above methods may be used for diagnosing disorders, diseases or pathological conditions in which the CD44vRA product is expressed at abnormal levels as compared to its expression in healthy individuals. In cases where the CD44vRA product is expressed only in abnormal conditions and not in healthy ones, the detection of its presence is sufficient to diagnose the abnormal condition. In cases where the variant product is expressed to some extent also in healthy individuals, normal standard expression values for the variant protein may be determined by obtaining body fluid samples from a number of healthy individuals and determining the level of expression of the variant product in a pool of these samples. The measured variable expression of the variant product in the tested individual may then be compared to the previously determined standard level.

The anti-CD44vRA antibodies of the invention may also be used as therapeutic agents for treatment of disorders, diseases or pathological conditions involving the expression of the CD44vRA product. Administration of the antibodies of the invention to an individual will result in blocking or decreasing the activity of the CD44vRA product and treatment of such disorders and diseases in which a beneficial effect can be achieved by such a decrease.

The antibodies of the invention will typically be administered within pharmaceutical compositions comprising one or more of the antibodies of the invention, preferably monoclonal antibodies, as active ingredients together with pharmaceutically acceptable carriers. The antibodies may be conjugated to various active agents such as radioactive molecules, enzymes, antibiotics or toxic agents and used as carriers to bring the agents into target cells comprising the CD44vRA product.

The above embodiments of the invention are preferably used for the identification, diagnosis and treatment of Rheumatoid arthritis (RNA).

### SPECIFIC EXAMPLES

### Materials and Methods

### Cloning and transfection of human CD44vRA, CD44v3-10 and CD44s

For cloning human CD44vRA cDNA, the total synovial fluid cell population of RA patients undergoing joint aspiration was isolated. RNA was separated with a commercial kit (Promega, Madison, WI), CD44vRA cDNA was prepared by RT-PCR (PTC-100™ Programmable Thermal Controller, MJ Research, Watertown, MA), using the following primers representing the constant coding regions of CD44 (Fig. 1A):
**Ex1**-sense, 5'-GAATTCGCCG CCACCATGGA CAAGTTTTGG TGG-3'; (SEQ ID NO:7).
**Ex19** - antisense, 5'-TCTAGATTAC ACCCCAATCT TCATG - 3'; (SEQ ID NO: 8);

PCR product size was confirmed by agarose gel electrophoresis and sequencing (ABI PRISM 310, Perkin-Elmer, Wellesley, MA) and Pstl (New England BioLabs, Beverly, MA) digestion (the nucleotide insertion in CD44vRA introduces a PstI digestion site).

The PCR product was excised from the gel, purified and subcloned into a pGEM vector (Promega). Positive clones were selected by white/blue screening. Plasmids were purified with a commercial kit (Promega), subjected in EcoRI/Xbal-double digestion and cloned into the pcDNA3.1 vector (Invitrogen, Paisley, UK) in which the gene product was expressed. The plasmid was transfected into the CD44-negative Namalwa Burkett lymphoma cell line (ATCC, Manassas, VA, ATCC No: CRL-1432) as described [Zhang Z et al. J. Biol. Chem. 276:41921-42929 (2001)]. For cloning of human CD44v3-10, RNA was isolated from human keratinocytes (Hadassah University Hospital, Jerusalem), and for cloning of human CD44s, RNA was isolated from the HeLa cervical cancer cell line (obtained from ATCC, Manassas, VA, ATCC No: CCL-2), using the above- described protocol.

Transfection of CD44v3-v10 and CD44s cDNAs as well as of the pcDNA3.1 vector ("empty vector") was performed as described above. Accordingly, the transfected Namalwa cells were designated *Namalwa-CD44vRA, Namalwa-CD44v3-v10, Namalwa-CD44s* and *Namalwa-Neo* (empty), respectively.

### Preparation of soluble hCD44v3-10, hCD44vRA and hCD44s plasmids

The soluble CD44v3-10 cDNA was cloned from total RNA of primary human keratinocyte by RT-PCR amplification, using two primers assigned from the published CD44 sequence;
**Ex1s**: 5'-TATCTAGAGC CGCCACCATG GACAAGTTTT GGTGG-3'; (SEQ ID NO:9)
**Ex16/17as**: 5'-TATCTAGAGCC ATTCTGGAAT TTGGGGTGT-3'; (SEQ ID NO:10)

Both primers contained a Xbal recognition site. The PCR products were digested with Xbal enzyme and pCXFc zeovector was digested with NheI. enzyme. After digestion, the PCR products were ligated into the pCXFc zeovector to generate CD44v3-v10-immunoglobulin (Ig)-Fc recombinant Using the same protocol, the soluble CD44vRA and soluble CD44s cDNAs were cloned from synovial cells of rheumatoid arthritis patients. The soluble CD44 fragments were assigned from the published sequence of CD44 (1-1824 bases) [Screaton, G.R., *et al.,* (1992) *ibid.*].

### Transient transfection of the soluble CD44 plasmids into 293T cells

A quantity of 3µg of each one of the above-indicated Fc containing plasmids was incubated for 20 min with 12 µl of FuGene (Roche). The mixture was added into 15 cm cell plates containing 70% confluent 293T cells (ATCC). Supernatant was collected after 48h and 72h. The CD44-Ig Fc fragmented proteoglycans were purified on protein-G column and analyzed for correctness by SDS-PAGE and immunoblotting with anti-pan-CD44 mAb (Hermes-3, ATCC Manassas, VA, ATCC No: HB-9480).

### Reverse transcriptase-polymerase chain reaction (RT-PCR)

RNA was extracted from synovial fluid cells of RA patients, primary human keratinocytes or Namalwa-CD44vRA cells, using RNA-BEE reagent (RNA isolation solvent, Tel-Test Inc., Friendswood, TX) according to the manufacturer's instructions. Reverse transcription was performed with 5 units of AMV reverse transcriptase (Promega, Medison, WI) in a 20 µl reaction volume containing 50 mM Tris-HCl, pH 8.3, 50 mM KCL, 10 mM MgCl₂, 10 mM dithiothreitol (DTT) and 20 units RNasin (Promega, Medison, WI), using 500 ng of RNA and 100 ng of oligo d(T)18 primer (Promega, Medison, WI). Reaction samples were incubated for 1 hour at 41°C and then the reverse transcriptase was inactivated by heating the mixture for 10 minutes at 65°C. The amplification was performed in a micro-processor-controlled incubator (MiniCycler^{™}, MJ Research, Watertown, MA), using 0.5 µl of the reverse transcriptase reaction product (cDNA) in a final volume of 50 µl containing 50 mM KCl, 1.5 mM MgCl₂, 10 mM Tris-HCl, pH 9.0, 250 µM dNTPs and 2.5 units Taq DNA polymerase (Promega, Medison, WI). The following primers (Fig. 1A) were added to reaction mixture, as also shown in Fig. 1A:
**hs5' sense**: 5'- GATGGAGAAAGCTCTGAGCATC - 3' (SEQ ID NO:11);
**pv31 sense**: 5'- ACGTCTTCAAATACCATCTC - 3' (SEQ ID NO:12);
**hs3' anti-sense**: 5'- TTTGCTCCACCTTCTTGACTCC - 3' (SEQ ID NO: 13);

The CD44 amplification was carried out for 30 cycles with denaturation at 94°C for 1 minute, annealing at 50°C for 1 minute and extension at 72°C for 2 minutes, followed by 10 minutes final extension at 72°C. The amplified products were resolved on 1.5% agarose gel. Determination of the cellular CD44 isoform transcripts was based on the position of the band in relation to the markers' ladder, and on the expected bp size of the different CD44 variants.

### Generation of monoclonal antibody secreting Hybridomas

A thirty mer CD44vRA peptide obtained from Corixa (Seattle, WA), SNPEVLLQTT TRMT**A**DVDRNGTTAYEGNWN (SEQ ID NO:14), and/or 100 µg/ml soluble CD44vRA produced as described above, both emulsified in complete Freund's adjuvant (CFA) (Sigma), were used to immunize subcutaneously or intramuscularly 8-week-old female C75BL/6 mice. The immunization was repeated on days 14 and 28 and two weeks later the mice were bled and their sera were tested by flow cytometry for their ability to bind to Namalwa cells expressing CD44. The animals with highest polyclonal anti-CD44 antibody titers were selected and boosted intraperitoneally (i.p.) with 10⁸ Namalwa-CD44vRA cells. After 72 h, spleen cells from the mice were harvested and fused with Myeloma cell line SP 2/0 myeloma cells according to Kohler and Milstein [Kohler, G., and Milstein C. Nature 256:495-497 (1975)].

After one day of incubation in enriched RPMI 1640 (Sigma) containing L-glutamine, penicillin-streptomycin solution, sodium pyruvate and MEM-eagle non-essential amino acids (Biological Industries Ltd., Israel) and 20% fetal bovine serum (FBS) (Sigma), the hybridomas were grown in ClonaCell™-HY Hybridoma Selection Medium (medium D, StemCell Technologies Inc.). Between days 10 to 14, isolated hybridoma colonies were collected from the semi-solid agar and grown in 96-well plates (Costar) in enriched RPMI 1640 medium containing HAT media supplement (Sigma) and 20% of FBS. At day 7 after plating, the supernatants from isolated hybridoma clones were screened by flow cytometry for their ability to bind to Namalwa-Neo, Namalwa-CD44v3-10 or Namalwa-CD44vRA cells. Hybridoma whose supernatants bound selectively or preferentially to Namalwa-CD44vRA were cloned by limiting dilution and then re-cloned for additional three cycles. The isolated hybridomas were maintained in enriched RPMI 1640 containing HAT media supplement and 20% FBS. The isotype of the CD44vRA-positive hybridoma supernatant was determined by ELISA musing Clonotype System-HRP (Southern Biotechnology Associates, Inc.).

### Fluorescence activated cell sorting (FACS) analysis

A quantity of 10⁶ cells were incubated with 3G5 anti-pan-CD44s (Hermes 3, IgG1) F-10-44-2 anti-pan-CD44 mAb (IgG2b, Serotec, Oxford, UK, known also as anti-CD44s mAb) or VFF7 anti-CD44v6 mAb (IgGl, Bender MedSystem, Vienna, Austria) for 45 min on ice. After extensive washing, the cells were incubated with fluorescein isothiocyanate (FITC)-conjugated secondary anti-Ig antibody (Jackson ImmunoResearch, West Grove, PA) for 30 min on ice. The cells were then washed and analyzed with a Flow Cytometer (Beckton Dickinson, San Jose, CA).

### Enzyme-linked immunosorbent assay (ELISA)

Polystyrene plates of 96 wells (Nunc) were coated with purified CD44vRA-IgFc, CD44v3-v10-IgFc, CD44s-IgFc soluble proteins or with BSA (100 µg/ml per well diluted in 100 µl sodium acetate buffer, pH 7.0). After overnight incubation at 4°C, the plates were washed three times with phosphate-buffered saline (PBS), pH 7.4, containing 0.05% Tween 20 (PBS/T). Following blocking with 10% milk in PBS at 37°C for 2 h, different concentrations of F8:33 anti-CD44vRA mAb or anti-pan-CD44 (Hermes 3) mAb were added to the wells. The plates were incubated at 37°C for 1 h, washed and a secondary goat anti-mouse polyvalent peroxidase-conjugated antibody (Jackson ImmunoResearch) was added for an additional 1 h. The enzyme reaction was developed with 0.04% H₂O₂ and 0.04% *O-*phenylenediamine in phosphate-citrate buffer, pH 5.0. The optical density was measured at 405 nm on a microplate reader MRX (Dynatech Laboratories) and values above 0.100 were considered positive.

### Western Blot analysis

Cells were lysed in NP-40 buffer and 100 µg of proteins were run on denaturing SDS-PAGE and transferred to a PVDF membrane (Millipore, Bedford, MA). Blots were blocked with 1% BSA in PBS containing 0.1% Tween-20 (PBS-T), and incubated for 1 h with 1 µg/ml Hermes-3 anti-pan-CD44 mAb, which was obtained from the ATCC hybridoma (ATCC No: HB-9480) supernatant and purified on a protein-G column. The blots were rewashed in PBS-T, incubated with the appropriate HRP-conjugated anti-Ig secondary antibody (1:10,000 dilution) (Jackson ImmunoResearch) for 45 min, rewashed in PBS-T and developed with ECL reagent (Amersham Biosciences, Buckinghamshire, UK).

### Transwell migration assay

Migration assays were performed in transwell plates (Costar, Cambridge, MA) of 6.5 mm diameter. The upper and lower compartments of the transwells were separated by a 5 µM pore polycarbonate filter coated overnight at 4°C with 0.5 mg/ml hyaluronic acid (HA; H1876, Sigma) in PBS, and then washed 3 times with RPMI 1640. A quantity of 5 × 10⁵ transfected Namalwa cells or human primary keratinocytes suspended in RPMI 1640 was added to the upper compartment and 293 T cell supernatant diluted in RPMI or stromal cell-derived factor-1 (SDF-1) (400 ng/ml diluted in RPMI; R&D systems) was added to the lower compartment. To evaluate the anti-migratory capacity of the antibody, F8:33 anti-CD44vRA mAb or anti-pan-CD44 (F10-44-2) mAb was added, in different concentrations, to the transwell plates, which were then incubated at 37°C with 5% CO₂ for 4 hours. Cells that migrated to the lower compartment were counted at the end of the incubation period by fluorescence-activated cell sorter (FACS; Becton Dickinson, San Jose CA) on high speed for one minute and values of cell number/min were recorded.

### Inhibition of Collagen-induced arthritis in mice

The following mAbs were used: rat anti-mouse CD44 constant region (rat, IgG2b) obtained from hybridoma KM81 (ATCC, TIB-241;22); rat anti-mouse cell surface immunoglobulin idiotype (rat, IgG2b) obtained from hybridoma 4D2 (provided by J. Haimovich, Tel-Aviv University, Maloney et. al., Hybridoma 4:191-209, 1985) was used as an irrelevant isotype control for KM81 mAb; mouse anti-human CD44vRA (mouse, IgG2a) obtained from hybridoma F8:33 (our developed hybridoma); mouse anti-human CD44 constant region (mouse, IgG2a) obtained from hybridoma F10-44-2 (was purchase from Serotec Company) was used as a non-biofunctional isotype control for F8:33 mAb.

In order to induce arthritis in mice, Male DBA/1 mice (8-12 weeks old) were injected intradermally at the base of the tail with 200 µg type II collagen purified from bovine articular cartilage and emulsified in complete Freund's adjuvant (CFA; Difco Laboratories, Detroit, MI, USA) as described in Williams et. al., Proc.Natl.Acad.Sci. USA 89:9784-9788, 1992. The mice received a booster injection of 200 µg type II collagen emulsified in CFA, 3 weeks after the first dose. The mice were inspected daily and each animal with erythema and/or swelling in one or more limbs was randomly assigned to one of 4 groups, which received intraperitoneal (i.p.) injections of KM81 anti-mouse CD44 mAb, 4D2 isotype matched control mAb, F8:33 anti-human CD44vRA mAb or F 10-44-2 non-biofunctional isotype control anti-human CD44 mAb. Each mouse was injected on the day of disease onset (day 0) and then every other day for 10 days with 200µg antibody in 100 µl PBS. Arthritis was monitored over the 10 days treatment period by measuring paw swelling.

In order to measure paw swelling, the thickness of each affected hind paw was measured with microcalipers. The results are expressed as a direct measure of paw width in millimeters.

### Statistical analysis

Data were analyzed using microcomputer programs for one-way ANOVA, followed by Student's t-test for unpaired values. *P*<0.05 was considered significant. The results are expressed as the mean ± s.e.m. Each experiment was repeated at least 3 times, all showing similar results.

### Results

### mRNA of CD44 expressed on synovial fluid cells of Rheumatoid Arthritis (RA) patients contains an intron-derived extra trinucleotide

Synovial fluid cells from RA patients were isolated following joint aspiration. Their total RNA was reverse transcribed and subjected to PCR, using primers representing the constant coding regions of CD44.

**Fig. 1B** shows the RT-PCR of synovial fluid cells derived from the joint of representative RA patients. Two major signals were detected: a fast- migrating band (571 bp) corresponds to CD44s, and a slow-migrating band (1714 bp) corresponds to CD44v3-v10, which is also expressed on keratinocytes. These findings were confirmed by direct sequencing (data not shown).

A CD44 variant was detected in synovial fluid cells derived from 52 of 55 RA patients and from 12 of 14 patients with psoriatic arthritis (PSA). Five of 12 samples from synovial fluid cells of osteoarthritis (OA) patients also displayed the CD44 variant.

The CD44 variant RT-PCR products from 29 of 55 RA patients and 8 of 14 PSA patients were sequenced. An extra trinucleotide (**CAG**) was detected between exon v4 and exon v5 (Fig. 1C) in 23 (of 29) RA patients and 7 (of 8) PSA patients following computerized alignment versus the wild type variant-CD44v3-v10 (Fig. 1C). The CAG trinucleotide was transcribed from the extreme end of the intron bridging exon v4 to exon v5, precisely at the splicing junction. This trinucleotide allows the translation of alanine (Ala) without interfering with the entire reading frame of CD44 transcript. Rheumatoid arthritis-derived CD44 variant with extra CAG was designated *CD44vRA.*

### Production of anti-CD44vRA mAb

Expression of extra Ala in CD44vRA appeared to induce a configuration change, allowing the generation of mAbs able to discriminate between the RA variant and the wild-type isoform-CD44v3-v10 (or the standard isoform CD44s). CD44vRA, CD44v3-v10 (derived from human keratinocytes) and CD44s (derived from HeLa cells) cDNAs were transfected into CD44-negative Namalwa Burkett lymphoma cell line. CD44 transfectants expressing high levels of CD44s, as well as CD44v3-v10 and CD44vRA transfectants expressing equal levels of v6-containing CD44 variant were selected (**Fig. 2A**). The transfectants were designated *Namalwa-CD44s, Namalwa-CD44v3-v10* and *Namalwa-CD44vRA,* respectively. Namalwa cells transfected with an empty vector were designated *Namalwa-Neo.*

C57BL/6 mice were immunized with soluble CD44vRA, incorporated into CFA and challenged with Namalwa-CD44vRA cells as described in, *Materials and Methods.* Splenocytes from mice showing polyclonal anti-CD4vRA antibodies in their serum were fused with SP2/0 myeloma cells. Hybridoma cell clones were selected according to the ability of their supernatants to bind to Namalwa-CD44vRA, but substantially not to Namalwa-CD44v3-v10, Namalwa-CD44s or to Namalwa-Neo, as indicated by flow cytometry. Clones and sub-clones were established from positive hybridoma cell colonies, and they were stable in culture for over 8 months. Anti-CD44vRA mAbs from supernatants of positive hybridomas, designated *F8:33,* were purified on G-protein column. Flow cytometry further revealed (**Figs. 2B**-**2E**) that at a concentration of 0.4 µg/ml, F8:33 anti-CD44vRA mAb interacted with Namalwa-CD44vRA, but not with the other transfectants, including the wild type-CD44v3-v10. At a concentration of 2 µg/ml or higher (Table 1) F8:33 cross-reacted with Namalwa-CD44v3-v10 (**Fig. 2B-2E**) and at even higher concentrations (>100 µg/ml) - with Namalwa-CD44s and Namalwa-Neo cells as well (not shown).

**Table 1: Binding of F8:33 anti-CD44vRA mAb to Cells**

| **F8:33 mAb Concentration** | **Namalwa-CD44vRA** | **Namalwa-CD44v3-10** | **RA synoviocytes** | **Keratinocytes** |
|---|---|---|---|---|
| **0.2µg/ml** | + | - | + | - |
| **0.4µg/ml** | + | - | + | - |
| **2µg/ml** | + | + | + | - |
| **4µg/ml** | + | + | + | - |
| **20µg/ml** | + | + | + | - |
| **40µg/ml** | N.D. | N.D. | + | - |
| **100µg/ml** | N.D. | N.D. | + | - |
| **200µg/ml** | + | + | + | + |

| | | | | |
|---|---|---|---|---|
| + binding; - no binding; **N.D.** not done | | | | |

The above findings were confirmed by ELISA showing that F8:33 mAb bound, in a dose-dependent manner, to CD44vRA-coated microwells at higher rates than to CD44v3-v10 or CD44s-coated microwells (**Fig. 3A**). In contrast, anti-pan CD44 mAb bound to a similar extent to CD44vRA and CD44v3-v10 (**Fig. 3B**), while it did not bind to CD44s, presumably due to its inability to recognize the relevant epitope. The identity of the soluble CD44 proteins was verified by Western Blot (**Fig. 3C**).

The antibodies bound to CD44vRA coated on microwells or expressed on Namalwa cells at higher rates than to the corresponding wild type molecule-CD44v3-v10 or to CD44s. Notably, CD44vRA and CD44v3-v10 are expressed to a similar extent on Namalwa cells, while CD44s is expressed on these cells at an even higher level, indicating that the preferential binding of F8:33 to Namalwa-CD44vRA is not quantitatively dictated. The selective binding of F8:33 anti-CD44vRA mAb to Namalwa-CD44vRA was detected at concentrations equal to or lower than 0.4 µg/ml. At increasing concentration, F8:33 first cross-reacts with Namalwa-CD44v3-v10 and then with Namalwa-CD44s, implying differential binding affinity, the highest to cell surface CD44vRA and the lowest to cell surface CD44s.

### Selective targeting of RA synoviocytes by F8:33 anti-CD44vRA mAb

The Namalwa transfectants are, in fact, an artificial model for evaluating the binding capacity and bioactivity of anti-CD44vRA mAbs. To obtain a more realistic assessment, the interaction of F8:33 with primary RA synoviocytes and primary keratinocytes was examined. Keratinocytes were chosen as a reference group, because they are known expressors of CD44v3-v10, the wild type of CD44vRA. Flow cytometry analysis revealed slightly higher expression of CD44s on synovial fluid cells of an RA patient than on keratinocytes derived from two donors, with variations in expression of v6-containing CD44. It was also noted that keratinocytes expressed v9-containing CD44 molecules (in which CD44v3-v10 is included) at much higher levels than RA synoviocytes (**Fig. 4A-4C**). Even so, at concentrations of 2 and 4 µg/ml, F8:33 anti-CD44vRA mAb interacted with synovial fluid cells, but not with keratinocytes as indicated by flow cytometry (**Fig. 4D-4E**). Even at as high a concentration as 100 µg/ml, F8:33 selectively bound to RA synoviocytes, while at a concentration of 200 µg/ml F8:33 cross-reacted with keratinocytes (Table 1). Synovial fluid cells were identified as CD44vRA-positive cells by Pstl digestion of their cDNA. Keratinocytes constitutively express CD44v3-v10.

### The CD44vRA-depetadence of transwell cell migration

The question whether the interaction between F8:33 anti-CD44vRA mAb and RA synoviocytes displays a bioactivity was investigated. To this end, the ability of F8:33 anti-CD44vRA to inhibit the migration of Namalwa transfectants as well as RA synovial fluid cells and keratinocytes in transwell migration assay was determined.

F8:3.3 anti-CD44vRA reduced, in a dose-dependent manner, the ability of Namalwa-CD44vRA cells to cross the HA-coated membrane more effectively than that of Namalwa-CD44v3-v10 (**Fig. 5A**). This antibody could not display any inhibitory effect on the migration of Namalwa-CD44s and Namalwa-Neo cells (at a concentration of 1 µg/ml, F8:33 enhanced the migration of Namalwa-Neo cells). Isotype-matched anti-pan-CD44 mAb (recognizing a constant epitope on all CD44 isoforms) slightly influenced, at the highest concentration only, the migration of all CD44 transfectants (**Fig. 5B**), implying the selective anti-migratory effect of F8:33.

F8:33 anti-CD44vRA mAb also reduced, in a dose-dependent manner, the ability of CD44vRA-positive RA synoviocytes, but not keratinocytes, to cross the HA-coated membrane as indicated by transwell migration assay (**Fig. 6A**). In contrast, the anti-pan-CD44 mAb did not interfere with the cell migration of both cell types (**Fig. 6B**).

F8:33-6-10-8 is also highly specific for the CD44vRA product (**Fig. 7A**-**7E**). The binding of antibodies derived from the F8:33-6-10-8 hybridoma were incubated with either Namalwa cells expressing the empty vector (Namalwa-pcDNA3.1), the standard CD44 (Namalwa-CD44std.), the CD44v3-v10 product (Namalwa-CD44v3-v10) or the CD44vRA product (Namalwa-CD44vRA). Already at 1.2 µg/ml (Fig. 7D), the F8:33-6-10-8 antibodies react specifically with the CD44vRA expressing cells, whereas they do not bind at all to the other cells. Even at 1.2 mg/ml (Fig. 7A), the F8:33-6-10-8 antibodies bind to the CD44vRA expressing cells with a higher affinity than to the other cells.

### Inhibition of collagen-induced arthritis in mice

The F8:33 mAbs were also tested using the *in vivo* assay of collagen-induced arthritis (CIA). Collagen-induced arthritis (CIA) is the animal analogue of rheumatoid arthritis (RA), a recurrent, systemic disease characterized by chronic inflammation within the joints, associated with synovitis and erosion of cartilage and bone.

As can be seen in **Fig. 8**, Collagen-induced arthritis (CIA) is inhibited by treatment with the anti-CD44vRA mAb F8:33, as indicated by reduction in paw swelling (PAW, mm). In contrast, the control antibody, anti-pan human CD44 monoclonal antibody F 10-44-2, was not able to reduce the arthritic activity.

As a positive control, an anti-pan mouse CD44 antibody, KM81, was used, while a mouse isotype-matched unrelated mAb, 4D2 monoclonal antibody, was used as a negative control (**Fig. 9**). As shown in Fig. 9, KM81 was also able to reduce paw swelling caused by CIA in mice, in contrast to the 4D2 monoclonal antibody.

To summarize, F8:33 is capable of *in vivo* biological function, as indicated by reducing arthritic activity in mice.

## Claims

1. A hybridoma cell having the depository Accession No. CNCM I-3015 (F8:33 hybridoma) or CNCM I-3016 (F8:33-6-8-10 hybridoma).

2. A monoclonal antibody (mAb) produced by the hybridoma cell of claim 1.

3. An antibody molecule being a Fab fragment or a F(ab')₂ fragment of the mAb of Claim 2.

4. A pharmaceutical composition comprising the antibody of 2 in combination with a pharmaceutically acceptable carrier.

5. The pharmaceutical composition of Claim 4, for the treatment of Rheumatoid Arthritis (RNA).

6. A method of purifying from a biological sample a polypeptide or protein containing an epitope recognized by the antibody of Claim 2, the method comprising:
(i) providing an affinity matrix comprising said antibody bound to a solid support;
(ii) contacting said biological sample with said affinity matrix, to produce an antibody-polypeptide complex and unbound biological sample;
(iii) removing the unbound biological sample; and
(iv) releasing the polypeptide from the antibody bound to the affinity matrix,

7. The method of Claim 6, wherein said polypeptide is CD44vRA polypeptide as set forth by SEQ ID NO:2,

8. A method for analyzing a sample for the presence of an antigen comprising:
(i) contacting the sample with the antibody of Claim 2, under conditions enabling the formation of a detectable complex of said antibody with an antigen; and
(ii) analyzing the data obtained from step (i) to see whether said antibody-antigen complex was formed, such formation indicating the presence in the sample of an antigen with an epitope recognized by said antibody.

9. A method for identifying a tested individual having a high probability of having a disease or disorder involving cells which express an antigen comprising an epitope recognized by the antibody of Claim 2, the method comprising:
(i) a biological sample from said tested individual;
(ii) contacting said biological sample with said antibody under conditions enabling the formation of a detectable antibody-antigen complex; and
(iii) detecting said antibody-antigen complex, the presence of said antibody-antigen complex indicating a high probability that the tested individual has a disease or disorder involving cells which express the CD44vRA protein as set forth by SEQ ID NO:2,

10. A method for identifying a tested individual having a high probability of having a disease or disorder involving cells which express an antigen comprising an epitope recognized by the antibody of Claim 2, the method comprising:
(i) a biological sample from said tested individual;
(ii) contacting said biological sample with said antibody under conditions enabling the formation of a detectable antibody-antigen complex; and
(iii) detecting said antibody-antigen complex and comparing the level of said complexes to the level of antibody-antigen complexes detected in a sample obtained from a healthy individual, a deviation from said level indicating a high probability that the tested individual has said disease or disorder.

11. The method of Claim 10, wherein said disease or disorder involves cells which express the CD44vRA as set forth by SEQ T17 NO:2.

12. The method of claim 9 or 10, wherein said disease is Rheumatoid Arthritis (RA).

13. Use of the pharmaceutical composition of claim 4 for the manufacture of a medicament for the treatment of Rheumatoid Arthritis (RA).

## Patentansprüche

1. Hybridomzelle mit der Hinterlegungsstellen-Zugangsnummer CNCM I-3015 (P8:33 Hybridom) oder CNCM I-3016 (F8:33-6-8-10 Hybridom).

2. Monoklonaler Antikörper (mAb), der durch die Hybridomzelle von Anspruch 1 erzeugt ist.

3. Antikürpermolekül, das ein Fab-Fragment oder ein F(ab')₂-Fragment des mAb von
Anspruch 2 ist.

4. Pharmazeutische Zusammensetzung, umfassend den Antikörper von Anspruch 2 in Kombination mit einem pharmazeutisch unbedenklichen Träger.

5. Pharmazeutische Zusammensetzung von Anspruch 4 für die Behandlung von rheumatoider Arthritis (RA).

6. Verfahren zum Reinigen aus einer biologischen Probe eines Polypeptids oder Proteins mit einem Epitop, das vom Antikörper von Anspruch 2 erkannt wird, wobei das Verfahren umfasst:
(i) Bereitstellen einer Affinitätsmatrix, die den Antikörper gebunden an einen festen Träger umfasst;
(ii) In-Kontakt-Bringen der biologischen Probe mit der Affinitätsmatrix, um einen Antikörper-Polypeptid-Komplex und eine ungebundene biologische Probe zu erzeugen;
(iii) Entfernen der ungebundenen biologischen Probe: und
(iv) Freisetzen des Polypeptids vom Antikörper, der an die Affinitätsmatrix gebunden ist.

7. Verfahren nach Anspruch 6, wobei das Polypeptid ein CD44vRA-Polypeptid ist, wie durch SEQ ID NO:2 dargelegt.

8. Verfahren zum Analysieren einer Probe auf das Vorhandensein eines Antigens, umfassend:
(i) In-Kontakt-Bringen der Probe mit dem Antikörper von Anspruch 2 unter Bedingungen, die die Bildung eines nachweisbaren Komplexes des Antikörpers mit einem Antigen ermöglichen; und
(ii) Analysieren der aus Schritt (i) erhaltenen Daten, um zu sehen, ob der Antikörper-Antigen-Komplex gebildet wurde, wobei eine derartige Bildung das Vorhandensein in der Probe eines Antigens mit einem Epitop anzeigt, das vom Antikörper erkannt wird,

9. Verfahren zum Identifizieren eines getesteten Individuums mit einer hohen Wahrscheinlichkeit, an einer Krankheit oder einer Störung zu leiden, an der Zellen beteiligt sind, die ein Antigen exprimieren, das ein Epitop umfasst, das vom Antikörper von Anspruch 2 erkannt wird, wobei das Verfahren Folgendes umfasst:
(i) eine biologische Probe des getesteten Individuums;
(i) In-Kontakt-Bringen der biologischen Probe mit dem Antikörper unter Bedingungen, die die Bildung eines nachweisbaren Antikörper-Antigen-Komplexes ermöglichen; und
(iii) Nachweisen des Antikörper-Antigen-Komplexes, wobei das Vorhandensein des Antiköiper-Antigen-Komplexes eine hohe Wahrscheinlichkeit anzeigt, dass das getestete Individuum an einer Krankheit oder Störung leidet, an der Zellen beteiligt sind, die das Cb44vRA-Protein exprimieren, wie durch SEQ ID NO:2 dargelegt.

10. Verfahren zum Identifizieren eines getesteten Individuum mit einer hohen Wahrscheinlichkeit, an einer Krankheit oder einer Störung zu leiden, an der Zellen beteiligt sind, die ein Antigen exprimieren, das ein Epitop umfasst, das vom Antikörper von Anspruch 2 erkannt wird, wobei das Verfahren Folgendes umfaßt:
(i) eine biologische Probe des getesteten Individuums;
(i) In-Kontakt-Bringen der biologischen Probe mit dem Antikörper unter Bedingungen, die die Bildung eines nachweisbaren Antikörper-Antigen-Komplexes ermöglichen; und
(iii) Nachweisen des Antikörper-Antigen-Komplexes und Vergleichen des Spiegels der Komplexe mit dem Spiegel von Antikörper-Antigen-Komplexen, die in einer von einem gesunden Individuum erhaltenen Probe nachgewiesen werden, wobei eine Abweichung vom Spiegel eine hohe Wahrscheinlichkeit anzeigt, dass das getestete Individuum an einer Krankheit oder einer Störung leidet.

11. Verfahren nach Anspruch 10 wobei an der Krankheit oder der Störung Zellen beteiligt sind, die das CD44vRA exprimieren, wie durch SEQ ID NO:2 dargelegt.

12. Verfahren nach Anspruch 9 oder 10, wobei die Krankheit rheumatoide Arthritis (RA) ist.

13. Verwendung der pharmazeutischen Zusammensetzung von Anspruch 4 für die I4erstellung eines Medikaments zur Behandlung von rheumatoider Arthritis (RA).

## Revendications

1. Cellule d'hybridome ayant le numéro d'accès de dépôt CNCM I-3015 (hybridome F8:33) ou CNCM I-3016 (hybridome F8:33-6-8-10).

2. Anticorps monoclonal (mAb) produit par la cellule d'hybridome selon la revendication 1.

3. Molécule d'anticorps qui est un fragment Fab ou un fragment F(ab')₂ du mAb selon la revendication 2.

4. Composition pharmaceutique comprenant l'anticorps selon la revendication 2, en combinaison avec un support pharmaceutiquement acceptable.

5. Composition pharmaceutique selon la revendication 4, destinée au traitement de la polyarthrite rhumatoïde (PR).

6. Procédé pour purifier, à partir d'un échantillon biologique, un polypeptide ou une protéine contenant un épitope reconnu par l'anticorps selon la revendication 2, le procédé consistant à :
(i) mettre à disposition une matrice d'affinité comprenant ledit anticorps lié à un support solide ;
(ii) mettre en contact ledit échantillon biologique avec ladite matrice d'affinité, afin de produire un complexe anticorps-polypeptide et un échantillon biologique non lié ;
(iii) éliminer l'échantillon biologique non lié ; et
(iv) libérer le polypeptide de l'anticorps lié à la matrice d'affinité.

7. Procédé selon la revendication 6, dans lequel ledit polypeptide est le polypeptide CD44vRA tel que décrit par SEQ ID NO: 2.

8. Procédé pour analyser un échantillon afin d'identifier la présence d'un antigène, consistant à :
(i) mettre en contact l'échantillon avec l'anticorps selon la revendication 2, dans des conditions qui permettent la formation d'un complexe détectable dudit anticorps avec un antigène ; et
(ii) analyser les données obtenues à l'étape (i) afin de vérifier si ledit complexe anticorps-antigéne s'est formé, une telle formation indiquant la présence dans l'échantillon d'un antigène porteur d'un épitope reconnu par ledit anticorps.

9. Procédé pour identifier un individu testé ayant une forte probabilité de présenter une maladie ou un trouble impliquant des cellules qui expriment un antigène comprenant un épitope reconnu par l'anticorps selon la revendication 2, le procédé comprenant :
(i) un échantillon biologique dudit individu testé ;
(ii) la mise en contact dudit échantillon biologique avec ledit anticorps dans des conditions qui permettent la formation d'un complexe anticorps-antigène détectable ; et
(iii) la détection dudit complexe anticorps-antigène, la présence dudit complexe anticorps--antigène indiquant une forte probabilité que l'individu testé présente une maladie ou un trouble impliquant des cellules qui expriment la protéine CD44vRA telle que décrite par SEQ ID NO: 2.

10. Procédé pour identifier un individu testé ayant une forte probabilité de présenter une maladie ou un trouble
impliquant des cellules qui expriment un antigène comprenant un épitope reconnu par l'anticorps selon la revendication 2, le procédé comprenant :
(i) un échantillon biologique dudit individu testé ;
(ii) la mise en contact dudit échantillon biologique avec ledit anticorps dans des conditions qui permettent la formation d'un complexe anticorps-antigène détectable ; et
(iii) la détection dudit complexe anticorps-antigène et la comparaison du taux desdits complexes au taux de complexes anticorps-antigène détectés dans un échantillon obtenu à partir d'un individu sain, une déviation par rapport audit taux indiquant une forte probabilité que l'individu testé présente ladite maladie ou ledit trouble.

11. Procédé selon la revendication 10, dans lequel ladite maladie ou ledit trouble implique des cellules qui expriment la CD44vRA telle que décrite par SEQ ID NO: 2.

12. Procédé selon la revendication 9 ou 10, dans lequel ladite maladie est la polyarthrite rhumatoïde (PR).

13. Utilisation de la composition pharmaceutique selon la revendication 4, pour la fabrication d'un médicament destiné au traitement de la polyarthrite rhumatoïde (PR).
